(19) Europäisches Patentamt
European Patent Office
Office européen des brevets

(11) EP 3 907 271 A1

(12) EUROPEAN PATENT APPLICATION

(43) Date of publication:
**10.11.2021 Bulletin 2021/45**

(51) Int Cl.:
***C11D 3/386*** *(2006.01)*

(21) Application number: **20173450.6**

(22) Date of filing: **07.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Novozymes A/S**
**2880 Bagsvaerd (DK)**

(72) Inventors:
- **HANSEN, Rasmus, Rune**
  **2880 Bagsvaerd (DK)**
- **ULBRINK, Inge, Byg**
  **2880 Bagsvaerd (DK)**

(74) Representative: **NZ EPO Representatives**
**Krogshoejvej 36**
**2880 Bagsvaerd (DK)**

# (54) CLEANING COMPOSITION, USE AND METHOD OF CLEANING

(57) The present invention relates to methods for cleaning a medical device, a cleaning composition, use of a polypeptide having DNase activity for cleaning a medical device and a medical device coated with a polypeptide having DNase activity.

EP 3 907 271 A1

## Description

### Field of the invention

The present invention relates to a method for cleaning a medical device, a medical cleaning composition, use of a polypeptide having DNase activity for cleaning a medical device and a medical device coated with a polypeptide having DNase activity.

### Background

Medical devices are often heavily contaminated with organic soil as a result of their use. Before re-use, it is essential that the devices are properly cleaned and disinfected.

The nature and extent of contamination in the healthcare environment is far greater than that normally found in a domestic environment, and the need for efficient cleaning is high. Of particular concern are microorganisms which are resistant to multiple types of antibiotics as these are over-represented in healthcare environments. Accordingly, use of unsufficiently reprocessed medical equipment poses a high risk of cross-infection to other patients, whose immune systems may already be compromised by illness, injury or the trauma of invasive medical procedures.

Typically, the procedure for reprocessing of medical devices comprises washing the device to remove organic materials, rinsing, disinfection and drying. Cleaned medical deviced are often still soiled with organic material, which can significantly decrease the efficacy of the subsequent disinfection procedure.

WO2017/129331 relates to cleaning of medical and dental instruments with protease. WO 2014/110015 relates to compositions comprising protease and methods for cleaning medical and dental instruments.

### Summary

The present invention pertains to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging.

In one aspect the invention relates to a medical cleaning composition comprising a polypeptide having DNase activity, wherein the composition has a pH in the range of 6.0-10.0.

In a further aspect the invention concerns use of a polypeptide having DNase activity for the cleaning at least part of a medical device.

In yet an aspect of the invention, the invention pertains to a medical device coated with a composition comprising a polypeptide having DNase activity.

### Overview of Figures

Figure 1: percent remaining dead biofilm after 60 minutes treatment with 0.5% model detergent with and without enzymes.

Figure 2: percent remaining dead biofilm from *P. aeruginosa* PA14 (grown for 4-hours or 24-hours before kill) after 60 minutes treatment with 0.5% model detergent and DNase in dosages from 0 microns/mL to 5 microns/mL.

Figure 3: percent remaining dead biofilm from *P. aeruginosa* PA01 (grown for 4-hours or 24-hours before kill) after 60 minutes treatment with 0.5% model detergent and DNase in dosages from 0 microns/mL to 5 microns/mL.

Figure 4: Absorbance of crystal violet stained dead biofilm from P. aeruginosa PA14 (grown for 4-hours before kill) after treatment with DNase in dosages from 0 - 5 ppm in endoscope reprocessing EU detergent 1, Dr. Weigert Neodisher Multizym.

Figure 5: Absorbance of crystal violet stained dead biofilm from P. aeruginosa PA14 (grown for 4-hours before kill) after treatment with DNase in dosages from 0 - 5 ppm in endoscope reprocessing EU detergent 2, Aniosyme Synergy 5.

Figure 6: Absorbance of crystal violet stained dead biofilm from P. aeruginosa PA14 (grown for 4-hours before kill) after treatment with DNase in dosages from 0 - 5 ppm in endoscope reprocessing US detergent 1, Steris Prolystica 2X Enzymatic Presoak and Cleaner.

Figure 7: Absorbance of crystal violet stained dead biofilm from P. aeruginosa PA14 (grown for 4-hours before kill) after treatment with DNase in dosages from 0 - 5 ppm in endoscope reprocessing US detergent 2, Metrex Metrizyme.

**Definitions**

By the term "at least the part of the medical device" is to be understood as the part of the device being in contact with the patient, or the interior of e.g. an endoscope which is in contact with fluids or samples, which has been in contact with the patient.

Medical cleaning composition: The term cleaning composition includes "detergent composition" and refers to compositions that find use in the removal of undesired compounds from items to be cleaned, such as medical devices. The terms encompass any materials/compounds selected for the particular type of cleaning composition desired and the form of the product (e.g., liquid, gel, powder, granulate, paste, or spray compositions). The medical cleaning composition may comprise one or more cleaning components.

Medical cleaning component: The medical cleaning component is a component different to the DNase enzyme. The precise nature of medical cleaning components, and levels of incorporation thereof, will depend on the physical form of the composition and the nature of the operation for which it is to be used. Suitable cleaning components include, but are not limited to, the components described below, under there respective headers. These includes wetting agents also known as surfactants, sequesting agents known as chelators or builders, which are used for removing water hardness, dispersants, polymeric agents, carriers, hydrotropes, processing aids, bactericides, fungicides, soil suspending agents, anti-corrosion agents, bluing agents and fluorescent dyes, antioxidants, and solubilizers.

In addition to containing the polypeptide having DNase, protease or mannanase activity, the medical cleaning composition may contain one or more additional enzymes (such as amylases, lipases, cutinases, cellulases, endoglucanases, xyloglucanases, pectinases, pectin lyases, xanthanases, peroxidaes, haloperoxygenases, catalases, galactanase or any mixture thereof), enzyme stabilizers and enzyme inhibitors, in particular protease inhibitors,.

Mature polypeptide: The term "mature polypeptide" means a polypeptide in its final form following translation and any post-translational modifications, such as N-terminal processing, C-terminal truncation, glycosylation, phosphorylation, etc.

Nomenclature: For purposes of the present invention, the nomenclature [E/Q] or [EQ] means that the amino acid at this position may be a glutamic acid (Glu, E) or a glutamine (Gln, Q). Likewise, the nomenclature [V/G/A/I] or [VGAI] means that the amino acid at this position may be a valine (Val, V), glycine (Gly, G), alanine (Ala, A) or isoleucine (Ile, I), and so forth for other combinations as described herein. Further, the amino acid X is defined such that it may be any of the 20 natural amino acids.

Sequence identity: The relatedness between two amino acid sequences or between two nucleotide sequences is described by the parameter "sequence identity".

For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows:

$$(\text{Identical Residues} \times 100)/(\text{Length of Alignment} - \text{Total Number of Gaps in Alignment})$$

The term "variant" means a polypeptide having enzyme activity comprising an alteration, *i.e.,* a substitution, insertion, and/or deletion, at one or more (*e.g.*, several) positions. A substitution means replacement of the amino acid occupying a position with a different amino acid; a deletion means removal of the amino acid occupying a position; and an insertion means adding an amino acid adjacent to and immediately following the amino acid occupying a position.

Wash liquor: The term "wash liquor" is defined herein as the solution or mixture of water and a cleaning

composition.

## DETAILED DESCRIPTION

In one aspect, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging.

Medical devices can be heavily soiled with organic soil. The organic soil may comprise material of organic origin, such as biofilm or material from a patient. The patient can be an animal or human being. The material may be of human or animal origin, e.g. combinations of organic material from animal or human, such as blood, mucus, tissue, adipose tissue, urine, faeces and microorganism e.g. bacteria. The organic material may adhere to the part of the medical device that has been into contact with the patient, or to the interior of e.g. an endoscope which is in contact with fluids or samples from the patient. The organic material may serve as nutrients for microorganisms present on the medical device or supplied from the use of the medical device on a patient. Microorganisms may produce a "biofilm", meaning a film produced by any group of microorganisms, in which the microbial cells stick to each other and/or stick to a surface of the medical device. These adherent microorganisms are frequently embedded within a self-produced matrix of extracellular polymeric substances (EPS). Biofilm EPS is a polymeric conglomeration generally composed of extracellular DNA, proteins, and polysaccharides. Biofilms may form on living or non-living surfaces such as the surface of a medical device. The microbial cells growing in a biofilm are physiologically distinct from planktonic cells of the same organism, which, by contrast, are single cells that may float or swim in a liquid medium. Microorganisms living in a biofilm usually have significantly different properties from planktonic bacteria of the same species, as the dense and protected environment of the film allows them to cooperate and interact in various ways. One benefit of this environment, for the microorganisms, is increased resistance to detergents and antibiotics, as the dense extracellular matrix and the outer layer of cells protect the interior of the community.

The organic soil may comprise one or more microorganisms, such as a group of microorganisms selected from the group consisting of, but not limited to *Acinetobacter sp., Aeromicrobium sp., Aspergillus versicolor, Brevundimonas sp., Enterobacter cloacae, Enterococcus faecalis, Escherichia coli, Klebsiella pneumoniae, Microbacterium sp., Micrococcus luteus, Pseudomonas sp., Staphylococcus aureus, Staphylococcus epidermidis, Stenotrophomonas sp., Bacillus sp. and Pseudomonas aeruginosa.* Especially *Pseudomonas aeruginosa* may be comprised in the organic soil.

In a preferred embodiment of the invention, the wash liquor comprises a polypeptide having DNase activity, and the polypeptides releases at least part of the organic soil present on the part of the medical device subjected to the wash liquor. In a preferred embodiment of the invention the polypeptides releases and/or removes at least part of the organic soil present on the part of the medical device subjected to the wash liquor.

Medical devices are typically disinfected by subjecting at least the part of the medical device soiled with organic soil to chemical, physical or biological disinfection e.g. to autoclaving, radiation or chemical disinfectants. However, if the cleaning step has been insufficient, the traditional methods for disinfection may leave organic soil on the surface of the device. The organic soil may comprise components from the patient, such as tissue or mucus, and microorganisms that are alive and/or dead.

In a preferred embodiment of the invention, the medical device is disinfected before being subjected to the wash liquor comprising the polypeptide having DNase activity. This situation occurs when the device has been used, unsufficiently cleaned, disinfected, used again and then are supposed to be cleaned. When an unsufficiently cleaned device is disinfected the remaining soil on said device will harbor an organic soil as described above. The disinfection may remove some or all of the organic soil or may leave organic soil on surfaces of the device. The disinfection may kill the microorganisms present in the organic soil, e.g. by killing microorganisms present in biofilm. Biofilm, in which all microorganisms are killed are termed dead biofilm. This dead biofilm will be subject to cleaning after the next use of the device.

In a preferred embodiment of the invention, a medical device soiled with a dead biofilm will be subject to cleaning in accordance with the method of the invention.

In a preferred embodiment of the invention, the disinfection is performed by subjecting at least part of the medical device to a chemical or a biological disinfection composition or to radiation. The disinfection may be performed by subjecting at least part of the medical device to antimicrobial agents, antibiotics, fungicides, oxidizing agents, hydrogen peroxide, chlorine, acids, peroxy acids, peracetic acid, potassium permanganate, ultra-violet radiation (UV) and/or ozon.

In a preferred embodiment of the invention, the disinfection leaves substantial no living microorganisms left in the organic soil present on the medical device.

In a preferred embodiment of the invention, the medical device is subjected to a liquid solution comprising peracetic acid having a pH of 3.0, whereafter no live microorganisms are comprised in the organic soil. The complete killing of microorganisms in organic soil and verification of the killing is demonstrated in example 1.

In a preferred embodiment of the invention, at least 80% of the microorganisms comprised in the organic soil are dead. In a more preferred embodiment of the invention, at least 85% of the microorganisms comprised in the organic soil are dead. In an even more preferred embodiment of the invention, at least 90% of the microorganisms comprised in the organic soil are dead. In a most preferred embodiment of the invention, at least 95% of the microorganisms comprised in the organic soil are dead. In a preferred embodiment of the invention 100% of the microorganisms in the organic soil are dead. Example 1 demonstrates how 100% of the microorganism present in the organic soil is dead.

In a preferred embodiment of the invention, the pH of the wash liquor may be in the range of 6.5-9.5. In a more preferred embodiment of the invention the pH of the wash liquor is in the range of 7.0-9.0, even more preferably in the range of 7.5-8.5, such as a pH around 8.

The wash liquor can be provided by mixing cleaning components with water and the polypeptide having DNase activity. In a preferred embodiment of the invention, the wash liquor is provided by mixing the medical cleaning composition of the invention with a suitable amount of liquid, e.g. water.

In an embodiment of the invention, the wash liquor comprises at least 0.001 ppm of the polypeptide having DNase activity. In a preferred embodiment, the wash liquor comprises at least 0.005 ppm of the polypeptide having DNase activity, more preferably at least 0.01 ppm of the polypeptide having DNase activity, even more preferably at least 0.05 ppm of the polypeptide having DNase activity or most preferably at least 1 ppm of the polypeptide having DNase activity.

In an embodiment of the invention, the wash liquor comprises at most 100 ppm of the polypeptide having DNase activity. In a preferred embodiment, the wash liquor comprises at most 50 ppm of the polypeptide having DNase activity, more preferably at most 30 ppm of the polypeptide having DNase activity, even more preferably at most 20 of the polypeptide having DNase activity or most preferably at most 10 ppm of the polypeptide having DNase activity.

In an embodiment of the invention, the wash liquor comprises in the range of 0.001-100 ppm of the polypeptide having DNase activity. In a preferred embodiment, the wash liquor comprises in the range of 0.005-50 ppm of the polypeptide having DNase activity, more preferably in the range of 0.01-30 ppm of the polypeptide having DNase activity, even more preferably in the range of 0.05-20 ppm of the polypeptide having DNase activity or most preferably in the range of 1-10 ppm more of the polypeptide having DNase activity.

In an embodiment of the invention, the wash liquor comprises in the range of 0.05-5 ppm of the polypeptide having DNase activity. In a preferred embodiment, the wash liquor comprises in the range of 0.025-5 ppm of the polypeptide having DNase activity, more preferably in the range of 0.05-5 ppm of the polypeptide having DNase activity, even more preferably in the range of 1.0-5 ppm of the polypeptide having DNase activity or most preferably in the range of 2.5-5 ppm more of the polypeptide having DNase activity.

The wash liquor may further comprise one or more cleaning composition components, preferably selected from surfactants, builders, polymers, dispersing agents and additional enzymes.

The additional enzymes may be selected from the group consisting of proteases, amylases, lipases, cutinases, cellulases, endoglucanases, xyloglucanases, pectinases, pectin lyases, xanthanases, peroxidaes, haloperoxygenases, catalases, galactanase or any mixture thereof.

In an embodiment of the invention, the wash liquor comprises in the range of 0.001-100 ppm of a polypeptide having mannanase activity. In a preferred embodiment, the wash liquor comprises in the range of 0.005-50 ppm of the polypeptide, more preferably in the range of 0.01-30 ppm of the polypeptide, even more preferably in the range of 0.05-20 ppm of the polypeptide or most preferably in the range of 1-10 ppm more of the polypeptide.

In an embodiment of the invention, the wash liquor comprises in the range of 0.001-100 ppm of a polypeptide having protease activity. In a preferred embodiment, the wash liquor comprises in the range of 0.005-50 ppm of the polypeptide, more preferably in the range of 0.01-30 ppm of the polypeptide, even more preferably in the range of 0.05-20 ppm of the polypeptide or most preferably in the range of 1-10 ppm more of the polypeptide.

In a preferred embodiment of the invention, the medical device is subjected to the wash liquor comprising the polypeptide having DNase activity. The medical device may comprise parts that do not tolerate to be subjected to a liquid, e.g. water or wash liquor. Thus, in some preferred embodiments of the invention the part of the medical device that does not tolerate liquid is not subjected to the wash liquor comprising the polypeptide having DNase activity. In some preferred embodiments of the invention, at least a part of the medical device is subjected to the wash liquor comprising the polypeptide having DNase activity, e.g. the part of the medical device that is in contact with the patient. In some preferred embodiments of the invention, the whole part of the medical device is subjected to the wash liquor comprising the polypeptide having DNase activity.

The medical device may be subjected to the wash liquor by spraying, dipping or immersing, e.g. by immersing

the whole part or at least part of the medical device into the wash liquor. In a preferred embodiment of the invention, the whole part or at least part of the medical device is soaked in the wash liquor for a time sufficient to reduce and/or remove soil on the instrument, preferably for at least 1 minute.

The medical device is soaked in the wash liquor for a time sufficient to be effective at reducing or removing the organic soil on the device. Depending on the level of soiling, the device may be soaked in the wash liquor for at least 1 minute. Soaking may continue even after cleaning is complete. For example, for convenience cleaning may continue over night, or even for up to 24 hours. The upper time limit is determined by the robustness of the device, to avoid damaging it. The medical device may be soaked in the wash liquor at a temperature between room temperature and 90 degrees Celsius, preferably between 20 and 90 degrees Celsius, more preferably between 30 and 80 degrees Celsius, even more preferably between 30 and 70 degrees Celsius, and most preferably between 30 and 60 degrees Celsius.

Soaking of the medical device may be carried out with or without mechanical action (such as shaking or stirring) in a tray, tub, pan, or sink; or by spraying, such as through an instrument washer; by ultrasonic treatment, treatment in a cart or cage washer; by manually applying it with a hand-held bottle as either a spray or a foam; or by mechanized washing in a laboratory glass machine washer.

In an embodiment, the cleaning of medical devices, and/or non-medical types of equipment, takes place in a (Medical) Washer-Disinfector according to EN ISO 15883-1 (or as described in "Class II Special Controls Guidance Document: Medical Washers and Medical Washer-Disinfectors; Guidance for the Medical Device Industry and FDA Review Staff", U.S. Food and Drug Administration, Feb. 2002), using the methods of the invention.

During soaking of the soiled medical device, at least part of the medical device is contacted with an effective amount of the wash liquor comprising the polypeptide having DNase activity. The actual amount of the medical cleaning composition used will be based on the judgment of the user and will depend upon factors such as the particular product formulation of the composition, the concentration of the composition, the number of soiled medical devices to be cleaned, and the degree of soiling of the articles. Subsequently, the items may be subjected to a manual or machine washing or rinsing method, involving either further washing steps and use of a cleaning composition, and/or to a manual or machine rinsing method. The disclosed compositions and methods are effective at removing the organic soil on devices in combination with mechanical action, such as e.g. scrubbing and may improve the cleaning process by reducing the amount of scrubbing required, and the overall result by a greater cleanliness of the medical device.

The wash liquor comprising the peptide having DNase activity can be employed in a variety of machines that clean medical devices. Such machines can be charged manually with liquid forms, or powder or other solid forms of the medical cleaning composition. Such machines can also automatically dispense the disclosed compositions. Such dispensing can include dissolving the medical cleaning composition to form a wash liquor, optionally diluting the wash liquor to yield a wash liquor (that is less concentrated) and diluting the second wash liquor into the wash or soak chamber to form the used wash liquor. The wash liquor can be used to clean the devices. Such dispensing can also include dissolving a solid cleaning composition once to form a wash liquor.

The medical device can be subjected to drying after being cleaned with the wash liquor comprising the peptide having DNase activity and/or being rinsed. In some embodiments, the medical device is packaged in a sterile package after being cleaned.

In an embodiment of the invention, the wash liquor comprising a polypeptide having DNase activity releases and/or removes at least 50% of the organic soil present on the part of the medical device subjected to the wash liquor. The released and/or removed organic soil can be measured as demonstrated in Example 2. In a preferred embodiment of the invention, preferably at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In an embodiment of the invention, the wash liquor comprising a polypeptide having DNase activity and an additional enzyme releases and/or removes at least 50% of the organic soil present (e.g. dead biofilm) on the part of the medical device subjected to the wash liquor. The released and/or removed organic soil can be measured as demonstrated in Example 1. In a preferred embodiment of the invention, preferably at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In a preferred embodiment of the invention, the invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,

d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In another preferred embodiment of the invention, the invention relates to a method for cleaning a medical device comprising the steps of:

a) Providing a wash liquor having a pH in the range of 6.0-10.0, comprising a polypeptide having DNase activity, a polypeptide having protease activity and/or a polypeptide having mannanase activity and optionally further cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In another preferred embodiment of the invention, the invention relates to a method for cleaning a medical device comprising the steps of:

a) Providing a wash liquor having a pH in the range of 6.0-10.0, comprising a polypeptide having DNase activity and a polypeptide having protease activity and/or a polypeptide having mannanase activity and optionally further cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In a preferred embodiment of the invention, the invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed and wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released

and/or removed, most preferably at least 90% of the organic soil is released and/or removed, such as at least 95% of the organic soil is released and/or removed.

In another preferred embodiment of the invention, the invention relates to a method for cleaning a medical device comprising the steps of:

a) Providing a wash liquor having a pH in the range of 6.0-10.0, comprising a polypeptide having DNase activity, a polypeptide of having protease activity and/or a polypeptide having mannanase activity and optionally further cleaning components,
b) Subjecting at least part of the medical device to the wash liquor of step a),
c) Subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

The medical device that are cleaned, according to the invention, include devices, instruments, or equipment, including any of the various medical or surgical instruments or devices that can benefit from cleaning with the wash liquor comprising the polypeptide having DNase activity. Exemplary medical devices include instruments, devices, tools, appliances, apparatus, and equipment used in medicine or surgery, including those than can be cold sterilized, soaked or washed and then heat sterilized, or otherwise benefit from cleaning in the disclosed compositions. These various instruments, devices and equipment include, but are not limited to: diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment, and the like, or combinations thereof. The medical device may be an indwelling device. The medical device can be selected from the group consisting of a catheter such as a central venous catheter, intravascular catheter, urinary catheter, Hickman catheter, peritoneal dialysis catheter, endrotracheal catheter, or wherein the device is a mechanical heart valve, a cardiac pacemaker, an arteriovenous shunt, a scleral buckle, a prosthetic joint, a tympanostomy tube, a tracheostomy tube, a voice prosthetic, a penile prosthetic, an artificial urinary sphincter, a synthetic pubovaginal sling, a surgical suture, a bone anchor, a bone screw, an intraocular lens, a contact lens, an intrauterine device, an aortofemoral graft, a vascular graft, a needle, a Luer-Lok connector, a needleless connector, surgical instrument, diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment.

In one embodiment of the invention, the invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is of bacterial or fungal origin.

In one embodiment of the invention, the invention relates to a method for cleaning a medical device comprising the steps of:

a) Providing a wash liquor having a pH in the range of 6.0-10.0, comprising a polypeptide having DNase activity, a polypeptide having protease activity and/or a polypeptide having mannanase activity and optionally further cleaning components,
b) Subjecting at least part of the medical device to the wash liquor of step a),
c) Optionally, subjecting the medical device to rinsing,

d) Optionally, subjecting the medical device to drying, and
e) Optionally, subjecting the medical device to sterile packaging,

wherein the polypeptide having DNase activity is of bacterial or fungal origin.

In one embodiment of the invention, the invention relates to a method for cleaning a medical device comprising the steps of:

a) Providing a wash liquor having a pH in the range of 6.0-10.0, comprising a polypeptide having DNase activity and/or a polypeptide having protease activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is of bacterial or fungal origin.

In one embodiment of the invention, the invention relates to a method for cleaning a medical device comprising the steps of:

a) Providing a wash liquor having a pH in the range of 6.0-10.0, comprising a polypeptide having DNase activity, and/or a polypeptide having mannanase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is of bacterial or fungal origin.

In a preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptides shown in SEQ ID NOs: 1, 2, 3, 4, 5, 6, 7, 8, 9 or 10, which have DNase activity and optionally comprising further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In a preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptide shown in SEQ ID NO: 1 which have DNase activity and optionally comprising further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical

device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.
In a preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptide shown in SEQ ID NO: 8 which have DNase activity and optionally comprising further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.
In another preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptides shown in SEQ ID NOs: 1, 2, 3, 4, 5, 6, 7, 8, 9 or 10, which have DNase activity, comprising a polypeptide having a sequence identity to the polypeptide shown in SEQ ID NO: 11 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity and optionally further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.
In another preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptides shown in SEQ ID NOs: 1, 2, 3, 4, 5, 6, 7, 8, 9 or 10, which have DNase activity, comprising a polypeptide having a sequence identity to the polypeptide shown in SEQ ID NO: 12 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity and optionally further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In another preferred embodiment of the invention, the invention relates to a method for cleaning a medical device comprising the steps of:

a) Providing a wash liquor having a pH in the range of 6.0-10.0, comprising a polypeptide having a sequence identity to the at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% to the polypeptides shown in SEQ ID NOs: 1, 2, 3, 4, 5, 6, 7, 8, 9 or 10, which have DNase activity, comprising a polypeptide having a sequence identity to the polypeptide shown in SEQ ID NO: 13, of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity and optionally further medical cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In another preferred embodiment of the invention, the invention relates to a method for cleaning a medical device comprising the steps of:

a) Providing a wash liquor having a pH in the range of 6.0-10.0, comprising a polypeptide selected from the group consisting of polypeptides comprising SEQ ID NOs: 1, 2, 3, 4, 5, 6, 7, 8, 9 and 10 and optionally further cleaning components,
b) Subjecting at least part of the medical device to the wash liquor of step a),
c) Optionally, subjecting the medical device to rinsing,
d) Optionally, subjecting the medical device to drying,
e) Optionally, subjecting the medical device to sterile packaging, and

wherein the wash liquor releases and/or removes at least 50% of the organic soil present on the part of the medical device subjected to the wash liquor. In a more preferred embodiment at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

In a further aspect, the invention concerns a medical cleaning composition comprising a polypeptide having DNase activity, wherein the composition has a pH in the range of 6.0-10.0.

The polypeptide having DNase activity can be obtained from Bacillus or Aspergillus. In a preferred embodiment of the invention, the polypeptide having DNase activity is obtained from Bacillus cibi or Aspergillus oryzae. The polypeptide having DNase activity may be used in the wash liquor or in the medical cleaning composition.

One embodiment relates to a medical cleaning composition comprising a polypeptide having DNase activity, wherein the composition preferably has a pH in the range of 6.0-10.0.

In a preferred embodiment of the invention, the medical cleaning composition and the medical cleaning method comprises a DNase.

Polypeptides having DNase activity are enzymes that catalyzes the hydrolytic cleavage of phosphodiester linkages in the DNA backbone, thus degrading DNA. The terms DNase and polypeptides having DNase activity can be used interchangeably. For purposes of the present invention, DNase activity is determined according to the procedure described in the Assay I.

In one embodiment, the polypeptides having DNase activity have at least 20%, e.g., at least 40%, at least 50%, at least 60%, at least 70%, at least 80%, at least 90%, at least 95%, or at least 100% of the DNase activity of the mature polypeptide of SEQ ID NO: 1, SEQ ID NO: 2, SEQ ID NO: 3, SEQ ID NO: 4, SEQ ID NO: 5, SEQ ID NO: 6, SEQ ID NO:

7, SEQ ID NO: 8, SEQ ID NO: 9 or SEQ ID NO: 10.

In one embodiment, the polypeptides having DNase activity have improved DNase activity, e.g. such that the DNase activity of the polypeptide is at least 105%, e.g., at least 110%, at least 120%, at least 130%, at least 140%, at least 160%, at least 170%, at least 180%, or at least 200% with reference to the DNase activity of the mature polypeptide of SEQ ID NOs: 1-10.

In a preferred embodiment of the invention, the polypeptide having DNase activity is selected from the group consisting of:

a) a polypeptide having at least 60%, at least 70%, at least 80%, at least 90%, at least 95%, or at least 100% sequence identity to the polypeptide shown in SEQ ID NO: 1,
b) a polypeptide having at least 60%, at least 70%, at least 80%, at least 90%, at least 95%, or at least 100% sequence identity to the polypeptide shown in SEQ ID NO: 2,
c) a polypeptide having at least 60%, at least 70%, at least 80%, at least 90%, at least 95%, or at least 100% sequence identity to the polypeptide shown in SEQ ID NO: 3,
d) a polypeptide having at least 60%, at least 70%, at least 80%, at least 90%, at least 95%, or at least 100% sequence identity to the polypeptide shown in SEQ ID NO: 4,
e) a polypeptide having at least 60%, at least 70%, at least 80%, at least 90%, at least 95%, or at least 100% sequence identity to the polypeptide shown in SEQ ID NO: 5,
f) a polypeptide having at least 60%, at least 70%, at least 80%, at least 90%, at least 95%, or at least 100% sequence identity to the polypeptide shown in SEQ ID NO: 6,
g) a polypeptide having at least 60%, at least 70%, at least 80%, at least 90%, at least 95%, or at least 100% sequence identity to the polypeptide shown in SEQ ID NO: 7,
h) a polypeptide having at least 60%, at least 70%, at least 80%, at least 90%, at least 95%, or at least 100% sequence identity to the polypeptide shown in SEQ ID NO: 8,
i) a polypeptide having at least 60%, at least 70%, at least 80%, at least 90%, at least 95%, or at least 100% sequence identity to the polypeptide shown in SEQ ID NO: 9 and
j) a polypeptide having at least 60%, at least 70%, at least 80%, at least 90%, at least 95%, or at least 100% sequence identity to the polypeptide shown in SEQ ID NO: 10.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 1.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 2.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 3.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 4.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature

polypeptide of SEQ ID NO: 5.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 6.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 7.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 8.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 9.

In a preferred embodiment, the present invention relates to a method for cleaning a medical device comprising a DNase, wherein the DNase having a sequence identity to the polypeptide of SEQ ID NO: 1 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 10.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,
wherein the polypeptide having DNase activity is

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1;
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 2;
iii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 3;
iv. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 4,
v. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 5,
vi. a polypeptide having at least 60% sequence, at least 65%, at least 70%, at least 75%, at least 80%, at least

85%, at least 90%, at least 91%, at least 92%, at least 93%, at least 94%, at least 95%, at least 96%, at least 97%, at least 98%, at least 99%, or 100% identity to the polypeptide shown in SEQ ID NO: 6,

vii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 7,

viii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8,

ix. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 9 or a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 10.

One embodiment relates to a medical cleaning composition comprising a polypeptide having protease activity, wherein the composition preferably has a pH in the range of 6.0-10.0.

In a preferred embodiment of the invention, the medical cleaning composition and the method of the invention comprises a protease.

The protease enzymes for use in the present invention include those of bacterial, fungal, plant, viral or animal origin e.g. vegetable or microbial origin. Microbial origin is preferred. Chemically modified or protein engineered mutants are included.

Preferably, the protease is a subtilisin. In the context of the present invention, the subtilisin enzyme family (EC 3.4.21.62) shall be understood as described by Siezen et al., Protein Engng. 4 (1991) 719-737 and Siezen et al. Protein Science 6 (1997) 501-523. As described therein, the subtilisin family may be divided into 3 sub-groups, i.e. I-S1 ("true" subtilisins), I-S2 (highly alkaline proteases) and intracellular subtilisins.

Examples of subtilisins are those derived from *Bacillus* such as *subtilisin lentus, Bacillus lentus, subtilisin Novo, subtilisin Carlsberg, Bacillus licheniformis, subtilisin BPN', subtilisin 309, subtilisin 147* and *subtilisin* 168 described in WO 89/06279 and protease PD138 (WO 93/18140). Additional examples are described in WO 98/020115, WO 01/44452, WO 01/58275, WO 01/58276, WO 03/006602 and WO 04/099401.

Examples of useful variants are described in WO 92/19729, WO 98/20115, WO 98/20116, and WO 98/34946, especially the variants with substitutions in one or more of the following positions: 3, 4, 9, 15, 27, 36, 57, 68, 76, 87, 95, 96, 97, 98, 99, 100, 101, 102, 103, 104, 106, 118, 120, 123, 128, 129, 130, 160, 167, 170, 194, 195, 199, 205, 206, 217, 218, 222, 224, 232, 235, 236, 245, 248, 252 and 274 using the BPN' numbering. More preferred the subtilase variants may comprise the mutations: S3T, V4I, S9R, A15T, K27R, *36D, V68A, N76D, N87S,R, *97E, A98S, S99G,D,A, S99AD, S101G,M,R S103A, V104I,Y,N, S106A, G118V,R, H120D,N, N123S, S128L, P129Q, S130A, G160D, Y167A, R170S, A194P, G195E, V199M, V205I, L217D, N218D, M222S, A232V, K235L, Q236H, Q245R, N252K, T274A (using BPN' numbering).

Examples of commercially available subtilisins include Kannase™, Everlase™, Primase, Duralase, Esperase™, Alcalase™, Durazym™, Savinase™, Ovozyme™, Liquanase™, Coronase™, Polarzyme™, Pyrase™, and Clear-Lens™ Pro; Blaze™ (all available from Novozymes A/S). Other commercially available proteases include Ronozyme™ Pro, Maxatase™, Maxacal™, Maxapem™, Opticlean™, Properase™, Purafect™, Purafect Ox™, Purafact Prime™, Excellase™, FN2™, FN3™ and FN4™ (available from Dupont).

In a preferred embodiment, the present invention relates to a method for cleaning a medical device comprising a protease, wherein the protease having a sequence identity to the polypeptide of SEQ ID NO: 11 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 11.

In a preferred embodiment, the present invention relates to a method for cleaning a medical device comprising a protease, wherein the protease having a sequence identity to the polypeptide shown in SEQ ID NO: 12 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have protease activity. In one aspect, the polypeptides differ by up to 10 amino acids, e.g., 1, 2, 3, 4, 5, 6, 7, 8, 9, or 10, from the mature polypeptide of SEQ ID NO: 12.

In a preferred embodiment, the present invention relates to a method for cleaning a medical device comprising the steps of:

a) Providing a wash liquor having a pH in the range of 6.0-10.0, comprising a polypeptide having protease activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,
wherein the polypeptide having protease activity is

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11 or
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.

In a preferred embodiment of the invention, a mannanase is used in the method of the invention or is comprised in the medical cleaning composition of the invention.
One embodiment relates to a medical cleaning composition comprising a polypeptide having mannanase activity, wherein the composition preferably has a pH in the range of 6.0-10.0.
In a preferred embodiment of the invention, the medical cleaning composition and the method of the invention comprises a mannanase.
The mannanase may be an alkaline mannanase of Family 5 or 26. It belongs It may be a wild type from *Bacillus* or *Humicola,* particularly *B. agaradhaerens, B. licheniformis, B. halodurans, B. clausii,* or *H. insolens.* Suitable mannanases are described in WO 99/064619. A commercially available mannanase is Mannaway™ (Novozymes A/S).
In a preferred embodiment, the present invention relates to a method for cleaning a medical device comprising a mannanase, wherein the mannanase having a sequence identity to the polypeptide shown in SEQ ID NO: 13 of at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100%, which have mannanase activity. In one aspect, the polypeptides differ by up to 10 amino acids, e.g., 1, 2, 3, 4, 5, 6, 7, 8, 9, or 10, from the mature polypeptide of SEQ ID NO: 13.
In a preferred embodiment, the present invention relates to a method for cleaning a medical device comprising the steps of:

a) Providing a wash liquor having a pH in the range of 6.0-10.0, comprising a polypeptide having mannanase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having mannase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.
In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1, and the wash liquor further comprises the a polypeptide having protease activity and having at least 60% sequence,

at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1, and the wash liquor further comprises the a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1, and the wash liquor further comprises the a polypeptide having mannanase activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8, and the wash liquor further comprises the a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11.

In a preferred embodiment, the present invention relates to a method for cleaning a medical device comprising the steps of:

a) Providing a wash liquor having a pH in the range of 6.0-10.0, comprising a polypeptide having DNase activity

and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8, and the wash liquor further comprises the a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8, and the wash liquor further comprises the a polypeptide having mannanase activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NOs: 1, 2, 3, 4, 5, 6, 7, 8, 9 or 10 and the wash liquor further comprises the a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least

70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NOs: 1, 2, 3, 4, 5, 6, 7, 8, 9 or 10 and the wash liquor further comprises the a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NOs: 1, 2, 3, 4, 5, 6, 7, 8, 9 or 10 and the wash liquor further comprises the a polypeptide having mannanase activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1, 2, 3, 4, 5, 6, 7, 8, 9 or 10 and the wash liquor further comprises a polypeptide having protease activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11, and a polypeptide having mannanase activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

In a preferred embodiment, the present invention relates to a method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging,

wherein the polypeptide having DNase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1, 2, 3, 4, 5, 6, 7, 8, 9 or 10 and the wash liquor further comprises a polypeptide having protease activity and having at least 60% sequence, at least 65%, at least 70%, at least 75%, at least 80%, at least 85%, at least 90%, at least 91%,

at least 92%, at least 93%, at least 94%, at least 95%, at least 96%, at least 97%, at least 98%, at least 99%, or 100% identity to the polypeptide shown in SEQ ID NO: 12, and a polypeptide having mannanase activity and having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying, and
e) Optionally, subjecting the medical device to sterile packaging,

preferably wherein the polypeptide having DNase activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1;
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 2;
iii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 3;
iv. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 4.
v. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 5,
vi. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 6,
vii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 7,
viii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8,
ix. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 9 or
x. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 10.

Preferably, the microorganisms comprised in the organic soil comprises *Pseudomonas aeruginosa.*

In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,

b) Subjecting at least part of the medical device to the wash liquor of step a),
c) Subjecting the medical device to rinsing,
d) Optionally, subjecting the medical device to drying, and
e) Optionally, subjecting the medical device to sterile packaging,
wherein the wash liquor further comprises a polypeptide having protease activity, wherein the polypeptide having protease activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11 or
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.

Preferably, the microorganisms comprised in the organic soil comprises *Pseudomonas aeruginosa.*
In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying, and
e) Optionally, subjecting the medical device to sterile packaging,
wherein the wash liquor further comprises a polypeptide having mannanase activity, wherein the polypeptide having mannanase activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.

Preferably, the microorganisms comprised in the organic soil comprises *Pseudomonas aeruginosa.*
In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying, and
e) Optionally, subjecting the medical device to sterile packaging,
wherein the polypeptide having DNase activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1;
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 2;
iii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 3;
iv. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least

97%, at least 98%, at least 99%, or 100% identity to the polypeptide shown in SEQ ID NO: 4;
v. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 5;
vi. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 6;
vii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 7;
viii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8;
ix. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 9 or
x. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 10,

wherein the wash liquor further comprises a polypeptide having protease activity, and wherein the polypeptide having protease activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 11.
Preferably, the microorganisms comprised in the organic soil comprises *Pseudomonas aeruginosa*.

In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying, and
e) Optionally, subjecting the medical device to sterile packaging,
wherein the polypeptide having DNase activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1;
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 2;
iii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 3;
iv. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 4;
v. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 5;
vi. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 6;
vii. a polypeptide having at least 60% sequence, at least 65%, at least 70%, at least 75%, at least 80%, at least

85%, at least 90%, at least 91%, at least 92%, at least 93%, at least 94%, at least 95%, at least 96%, at least 97%, at least 98%, at least 99%, or 100% identity to the polypeptide shown in SEQ ID NO: 7;
viii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8;
ix. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 9 or
x. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 10,

wherein the wash liquor further comprises a polypeptide having protease activity, and wherein the polypeptide having protease activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 12.
Preferably, the microorganisms comprised in the organic soil comprises *Pseudomonas aeruginosa.*
In a preferred embodiment, the present invention relates to a method for cleaning a medical device, wherein at least a part of the medical device comprises organic soil comprising one ore more microorganisms, which part has been subjected to a liquid solution comprising peracetic acid having a pH of 3, whereafter no live microorganisms are comprised in the organic soil, the method comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Subjecting the medical device to rinsing,
d) Optionally, subjecting the medical device to drying, and
e) Optionally, subjecting the medical device to sterile packaging,

wherein the polypeptide having DNase activity is:

i. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 1;
ii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 2;
iii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 3;
iv. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 4;
v. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 5;
vi. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 6;
vii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 7;
viii. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 8;
ix. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 9 or

x. a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 10,

wherein the wash liquor further comprises a polypeptide having mannanse activity, and wherein the polypeptide having mannanase activity is a polypeptide having at least 60% sequence, at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% identity to the polypeptide shown in SEQ ID NO: 13.
Preferably, the microorganisms comprised in the organic soil comprises *Pseudomonas aeruginosa.*

In one aspect of the invention, a polypeptide having DNase activity is used for releasing and/or removing organic soil from a medical device.

The use for medical cleaning may be indwelling medical device characterized in that at least a portion of a patient-contactable surface of the device is coated with a composition comprising a polypeptide having DNase activity.

In one aspect of the invention, a polypeptide having DNase activity and a polypeptide having protease activity are used for releasing and/or removing organic soil from a medical device.

The use for medical cleaning may be indwelling medical device characterized in that at least a portion of a patient-contactable surface of the device is coated with a composition comprising a polypeptide having DNase activity and a polypeptide having protease activity.

In one aspect of the invention, a polypeptide having DNase activity and a polypeptide having mannanase activity are used for releasing and/or removing organic soil from a medical device.

The use for medical cleaning may be indwelling medical device characterized in that at least a portion of a patient-contactable surface of the device is coated with a composition comprising a polypeptide having DNase activity and a polypeptide having mannanase activity.

In one aspect of the invention, a polypeptide having DNase activity, a polypeptide having protease activity and a polypeptide having mannanase activity are used for releasing and/or removing organic soil from a medical device.

The use for medical cleaning may be indwelling medical device characterized in that at least a portion of a patient-contactable surface of the device is coated with a composition comprising a polypeptide having DNase activity, a polypeptide having protease activity and a polypeptide having mannanase activity.

The device may be a catheter such as a central venous catheter, intravascular catheter, urinary catheter, Hickman catheter, peritoneal dialysis catheter, endrotracheal catheter, or wherein the device is a mechanical heart valve, a cardiac pacemaker, an arteriovenous shunt, a scleral buckle, a prosthetic joint, a tympanostomy tube, a tracheostomy tube, a voice prosthetic, a penile prosthetic, an artificial urinary sphincter, a synthetic pubovaginal sling, a surgical suture, a bone anchor, a bone screw, an intraocular lens, a contact lens, an intrauterine device, an aortofemoral graft, a vascular graft, a needle, a Luer-Lok connector, a needleless connector, surgical instrument, diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment.

The medical device may be characterized in that at least a portion of a patient-contactable surface of the device is coated with composition comprising a polypeptide having DNase activity, a polypeptide having protease activity and/or polypeptide having mannanse activity. The medical device may be selected from the group consisting of a catheter such as a central venous catheter, intravascular catheter, urinary catheter, Hickman catheter, peritoneal dialysis catheter, endrotracheal catheter, or wherein the device is a mechanical heart valve, a cardiac pacemaker, an arteriovenous shunt, a scleral buckle, a prosthetic joint, a tympanostomy tube, a tracheostomy tube, a voice prosthetic, a penile prosthetic, an artificial urinary sphincter, a synthetic pubovaginal sling, a surgical suture, a bone anchor, a bone screw, an intraocular lens, a contact lens, an intrauterine device, an aortofemoral graft, a vascular graft, a needle, a Luer-Lok connector, a needleless connector, surgical instrument, diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment.

In one aspect of the invention, the invention concerns a medical device coated with a composition comprising a polypeptide having DNase activity, a polypeptide having protease activity and/or polypeptide having mannanse activity.

In an embodiment of the invention, the medical cleaning composition is in the form of a bar, a homogenous tablet, a tablet having two or more layers, a pouch having one or more compartments, a regular or compact powder, a granule, a paste, a gel, a regular liquid, a compact liquid or concentrated liquid.

The liquid medical cleaning composition has a physical form, which is not solid (or gas). It may be a pourable liquid, a pourable gel or a non-pourable gel. It may be isotropic or structured, preferably isotropic. It may be a formulation

useful for washing in automatic washing machines or for hand washing.

The liquid medical cleaning composition may be aqueous, typically containing at least 20% by weight and up to 95% water, such as up to 70% water, up to 50% water, up to 40% water, up to 30% water, or up to 20% water. Other types of liquids, including without limitation, alkanols, amines, diols, ethers and polyols may be included in an aqueous liquid detergent. An aqueous liquid detergent may contain from 0-30% organic solvent. A liquid medical cleaning composition may even be non-aqueous, wherein the water content is below 10%, preferably below 5%.

Medical cleaning components can be separated physically from each other by compartments in water dissolvable pouches. Thereby negative storage interaction between components can be avoided. Different dissolution profiles of each of the compartments can also give rise to delayed dissolution of selected components in the wash solution.

In addition to the polypeptides having DNase, protease or mannanase activity, the medical cleaning composition may comprise additional enzymes as well as medical cleaning components. The choice of additional components is within the skill of the artisan and includes conventional ingredients, including the exemplary non-limiting components set forth below. In a preferred embodiment of the invention the medical cleaning composition comprises additional enzymes.

In a preferred embodiment of the invention, a lipase is used in the method of the invention or is comprised in the medical cleaning composition of the invention.

Suitable lipases and cutinases include those of bacterial or fungal origin. Chemically modified or protein engineered mutants are included. Examples include lipase from *Thermomyces, e.g.,* from *T. lanuginosus* (previously named *Humicola lanuginosa*) as described in EP 258068 and EP 305216, cutinase from *Humicola, e.g. H. insolens* as described in WO 96/13580, a *Pseudomonas* lipase, *e.g.,* from *P. alcaligenes* or *P. pseudoalcaligenes* (EP 218272), *P. cepacia* (EP 331376), *P. stutzeri* (GB 1372034), *P. fluorescens, Pseudomonas sp.* strain SD 705 (WO 95/06720 and WO 96/27002), *P. wisconsinensis* (WO 96/12012), a *Bacillus* lipase, *e.g.,* from B. *subtilis* (Dartois et al., Biochemica et Biophysica Acta, (1993), 1131, 253-360), *B. stearothermophilus* (JP 64/744992) or *B. pumilus* (WO 91/16422), GDSL-type *Streptomyces* lipases (WO10/065455), cutinase from *Magnaporthe grisea* (WO10/107560), cutinase from *Pseudomonas mendocina* (US5,389,536), lipase from *Thermobifida fusca* (WO11/084412), *Geobacillus stearothermophilus* lipase (WO11/084417), lipase from *Bacillus subtilis* (WO11/084599), and lipase from *Streptomyces griseus* (WO11/150157) and S. *pristinaespiralis* (WO12/137147).

Other examples are lipase variants such as those described in WO 92/05249, WO 94/01541, EP 407225, EP 260105, WO 95/35381, WO 96/00292, WO 95/30744, WO 94/25578, WO 95/14783, WO 95/22615, WO 97/04079, WO 97/07202, WO 00/060063, WO 07/087508 and WO 09/109500.

Preferred commercially available lipase enzymes include Lipolase™, Lipolase Ultra™, and Lipex™; Lecitase™, Lipolex™; Lipoclean™, Lipoprime™ (Novozymes A/S). Other commercially available lipases include Lumafast (Genencor Int Inc); Lipomax (Gist-Brocades/Genencor Int Inc) and Bacillus sp lipase from Solvay.

In a preferred embodiment of the invention, a carbohydrase is used in the method of the invention or is comprised in the medical cleaning composition of the invention.

A carbohydrase is a general term for enzymes that cleave carbohydrates. In general carbohydrases are named after the substrates they act on, for example amylases act on amylase and cellulases act on cellulose. Many carbohydrases have found use in cleaning and laundry applications, such as amylase, cellulase, pectinase, pectate lyase, mannanase, arabinase, galactanase and xylanase, and all these can be applied in the liquid cleaning composition.

In a preferred embodiment of the invention, an amylase is used in the method of the invention or is comprised in the medical cleaning composition of the invention.

Suitable amylases include those of bacterial or fungal origin. Chemically modified or protein engineered mutants are included. Amylases include, for example, a-amylases obtained from *Bacillus, e.g.,* a special strain of *Bacillus licheniformis,* described in more detail in GB 1,296,839.

Examples of suitable amylases include amylases having SEQ ID NO: 2 in WO 95/10603 or variants having 90% sequence identity to SEQ ID NO: 3 thereof. Preferred variants are described in WO 94/02597, WO 94/18314, WO 97/43424 and SEQ ID NO: 4 of WO 99/019467, such as variants with substitutions in one or more of the following positions: 15, 23, 105, 106, 124, 128, 133, 154, 156, 178, 179, 181, 188, 190, 197, 201, 202, 207, 208, 209, 211, 243, 264, 304, 305, 391, 408, and 444.

Different suitable amylases include amylases having SEQ ID NO: 6 in WO 02/010355 or variants thereof having 90% sequence identity to SEQ ID NO: 6. Preferred variants of SEQ ID NO: 6 are those having a deletion in positions 181 and 182 and a substitution in position 193. Other amylases which are suitable are hybrid alpha-amylase comprising residues 1-33 of the alpha-amylase derived from *B. amyloliquefaciens* shown in SEQ ID NO: 6 of WO 2006/066594 and residues 36-483 of the *B. licheniformis* alpha-amylase shown in SEQ ID NO: 4 of WO 2006/066594 or variants having 90% sequence identity thereof. Preferred variants of this hybrid alpha-amylase are those having a substitution, a deletion or an insertion in one of more of the following positions: G48, T49, G107, H156, A181, N190, M197, 1201, A209 and Q264. Most preferred variants of the hybrid alpha-amylase comprising residues 1-33 of the alpha-amylase derived from *B. amyloliquefaciens* shown in SEQ ID NO: 6 of WO 2006/066594 and residues 36-483 of SEQ ID NO: 4 are those

having the substitutions:

M197T;
H156Y+A181T+N190F+A209V+Q264S; or
G48A+T49I+G107A+H156Y+A181T+N190F+I201F+A209V+Q264S.

Further amylases which are suitable are amylases having SEQ ID NO: 6 in WO 99/019467 or variants thereof having 90% sequence identity to SEQ ID NO: 6. Preferred variants of SEQ ID NO: 6 are those having a substitution, a deletion or an insertion in one or more of the following positions: R181, G182, H183, G184, N195, I206, E212, E216 and K269. Particularly preferred amylases are those having deletion in positions R181 and G182, or positions H183 and G184.

Additional amylases which can be used are those having SEQ ID NO: 1, SEQ ID NO: 3, SEQ ID NO: 2 or SEQ ID NO: 7 of WO 96/023873 or variants thereof having 90% sequence identity to SEQ ID NO: 1, SEQ ID NO: 2, SEQ ID NO: 3 or SEQ ID NO: 7. Preferred variants of SEQ ID NO: 1, SEQ ID NO: 2, SEQ ID NO: 3 or SEQ ID NO: 7 are those having a substitution, a deletion or an insertion in one or more of the following positions: 140, 181, 182, 183, 184, 195, 206, 212, 243, 260, 269, 304 and 476. More preferred variants are those having a deletion in positions 181 and 182 or positions 183 and 184. Most preferred amylase variants of SEQ ID NO: 1, SEQ ID NO: 2 or SEQ ID NO: 7 are those having a deletion in positions 183 and 184 and a substitution in one or more of positions 140, 195, 206, 243, 260, 304 and 476.

Other amylases which can be used are amylases having SEQ ID NO: 2 of WO 08/153815, SEQ ID NO: 10 in WO 01/66712 or variants thereof having 90% sequence identity to SEQ ID NO: 2 of WO 08/153815 or 90% sequence identity to SEQ ID NO: 10 in WO 01/66712. Preferred variants of SEQ ID NO: 10 in WO 01/66712 are those having a substitution, a deletion or an insertion in one of more of the following positions: 176, 177, 178, 179, 190, 201, 207, 211 and 264.

Further suitable amylases are amylases having SEQ ID NO: 2 of WO 09/061380 or variants having 90% sequence identity to SEQ ID NO: 2 thereof. Preferred variants of SEQ ID NO: 2 are those having a truncation of the C-terminus and/or a substitution, a deletion or an insertion in one of more of the following positions: Q87, Q98, S125, N128, T131, T165, K178, R180, S181, T182, G183, M201, F202, N225, S243, N272, N282, Y305, R309, D319, Q320, Q359, K444 and G475. More preferred variants of SEQ ID NO: 2 are those having the substitution in one of more of the following positions: Q87E,R, Q98R, S125A, N128C, T131I, T165I, K178L, T182G, M201L, F202Y, N225E,R, N272E,R, S243Q,A,E,D, Y305R, R309A, Q320R, Q359E, K444E and G475K and/or deletion in position R180 and/or S181 or of T182 and/or G183. Most preferred amylase variants of SEQ ID NO: 2 are those having the substitutions:

N128C+K178L+T182G+Y305R+G475K;
N128C+K178L+T182G+F202Y+Y305R+D319T+G475K;
S125A+N128C+K178L+T182G+Y305R+G475K; or
S125A+N128C+T131I+T165I+K178L+T182G+Y305R+G475K wherein the variants are C-terminally truncated and optionally further comprises a substitution at position 243 and/or a deletion at position 180 and/or position 181.

Other suitable amylases are the alpha-amylase having SEQ ID NO: 12 in WO01/66712 or a variant having at least 90% sequence identity to SEQ ID NO: 12. Preferred amylase variants are those having a substitution, a deletion or an insertion in one of more of the following positions of SEQ ID NO: 12 in WO01/66712: R28, R118, N174; R181, G182, D183, G184, G186, W189, N195, M202, Y298, N299, K302, S303, N306, R310, N314; R320, H324, E345, Y396, R400, W439, R444, N445, K446, Q449, R458, N471, N484. Particularly preferred amylases include variants having a deletion of D183 and G184 and having the substitutions R118K, N195F, R320K and R458K, and a variant additionally having substitutions in one or more position selected from the group: M9, G149, G182, G186, M202, T257, Y295, N299, M323, E345 and A339, most preferred a variant that additionally has substitutions in all these positions.

Other examples are amylase variants such as those described in WO2011/098531, WO2013/001078 and WO2013/001087.

Commercially available amylases are Stainzyme™, Stainzyme Plus™, Amplify™, Resilience™, Everest™, Duramyl™, Termamyl™, Termamyl Ultra™; Natalase™, Fungamyl™ and BAN™ (Novozymes A/S), Rapidase™ and Purastar™/Effectenz™, Powerase™ and Preferenz S100 (from Genencor International Inc./DuPont).

In a preferred embodiment of the invention, a lyase is used in the method of the invention or is comprised in the medical cleaning composition of the invention.

The lyase may be a pectate lyase derived from *Bacillus,* particularly *B. licherniformis* or *B. agaradhaerens,* or a variant derived of any of these, e.g. as described in US 6124127, WO 99/027083, WO 99/027084, WO 02/006442, WO 02/092741, WO 03/095638, Commercially available pectate lyases are XPect™, Pectawash™, and Pectaway™ (Novozymes A/S).

In a preferred embodiment of the invention, a cellulase is used in the method of the invention or is comprised in the medical cleaning composition of the invention.

Suitable cellulases may be of bacterial or fungal origin. Chemically or genetically modified mutants are included. Suitable cellulases include cellulases from the genera *Bacillus, Pseudomonas, Humicola, Fusarium, Thielavia, Acremonium, e.g.,* the fungal cellulases produced from *Humicola insolens, Myceliophthora thermophila* and *Fusarium oxysporum* disclosed in US 4,435,307, US 5,648,263, US 5,691,178, US 5,776,757 and WO 89/09259.

Especially suitable cellulases are the alkaline or neutral cellulases having color care benefits. Examples of such cellulases are cellulases described in EP 0 495 257, EP 0 531 372, WO 96/11262, WO 96/29397, WO 98/08940. Other examples are cellulase variants such as those described in WO 94/07998, EP 0 531 315, US 5,457,046, US 5,686,593, US

Commercially available cellulases include Carezyme™, Celluzyme™, Celluclean™, Celluclast™, Endolase™, Renozyme™, Whitezyme™ (Novozymes A/S); Clazinase™, Puradax, Puradax HA, and Puradax EG (available from Genencor) and KAC-500(B)™ (Kao Corporation).

In a preferred embodiment of the invention, a peroxidase is used in the method of the invention or is comprised in the medical cleaning composition of the invention.

Suitable peroxidases are comprised by the enzyme classification EC 1.11.1.7, as set out by the Nomenclature Committee of the International Union of Biochemistry and Molecular Biology (IUBMB), or any fragment derived therefrom, exhibiting peroxidase activity.

Suitable peroxidases include those of plant, bacterial or fungal origin. Chemically modified or protein engineered mutants are included. Examples of useful peroxidases include peroxidases from *Coprinopsis, e.g.,* from *C. cinerea* (EP 179,486), and variants thereof as those described in WO 93/24618, WO 95/10602, and WO 98/15257.

The peroxidases also include a haloperoxidase enzyme, such as chloroperoxidase, bromoperoxidase and compounds exhibiting chloroperoxidase or bromoperoxidase activity. Haloperoxidases are classified according to their specificity for halide ions. Chloroperoxidases (E.C. 1.11.1.10) catalyze formation of hypochlorite from chloride ions.

In an embodiment, the haloperoxidase of the invention is a chloroperoxidase. Preferably, the haloperoxidase is a vanadium haloperoxidase, *i.e.,* a vanadate-containing haloperoxidase. In a preferred method of the present invention, the vanadate-containing haloperoxidase is combined with a source of chloride ion.

Haloperoxidases have been isolated from many different fungi, in particular from the fungus group dematiaceous hyphomycetes, such as *Caldariomyces, e.g., C. fumago, Alternaria, Curvularia, e.g., C. verruculosa* and *C. inaequalis, Drechslera, Ulocladium* and *Botrytis.*

Haloperoxidases have also been isolated from bacteria such as *Pseudomonas, e.g., P. pyrrocinia* and *Streptomyces, e.g., S. aureofaciens.*

In an preferred embodiment, the haloperoxidase is derivable from *Curvularia* sp., in particular *Curvularia verruculosa* or *Curvularia inaequalis,* such as *C. inaequalis* CBS 102.42 as described in WO 95/27046; or *C. verruculosa* CBS 147.63 or *C. verruculosa* CBS 444.70 as described in WO 97/04102; or from *Drechslera hartlebii* as described in WO 01/79459, *Dendryphiella salina* as described in WO 01/79458, *Phaeotrichoconis crotalarie* as described in WO 01/79461, or *Geniculosporium* sp. as described in WO 01/79460.

Suitable oxidases include, in particular, any laccase enzyme comprised by the enzyme classification EC 1.10.3.2, or any fragment derived therefrom exhibiting laccase activity, or a compound exhibiting a similar activity, such as a catechol oxidase (EC 1.10.3.1), an o-aminophenol oxidase (EC 1.10.3.4), or a bilirubin oxidase (EC 1.3.3.5).

Preferred laccase enzymes are enzymes of microbial origin. The enzymes may be derived from plants, bacteria or fungi (including filamentous fungi and yeasts).

Suitable examples from fungi include a laccase derivable from a strain of *Aspergillus, Neurospora, e.g., N. crassa, Podospora, Botrytis, Collybia, Fomes, Lentinus, Pleurotus, Trametes, e.g., T. villosa* and *T. versicolor, Rhizoctonia, e.g., R. solani, Coprinopsis, e.g., C. cinerea, C. comatus, C. friesii,* and *C. plicatilis, Psathyrella, e.g., P. condelleana, Panaeolus, e.g., P. papilionaceus, Myceliophthora, e.g., M. thermophila, Schytalidium, e.g., S. thermophilum, Polyporus, e.g., P. pinsitus, Phlebia, e.g., P. radiata* (WO 92/01046), or *Coriolus, e.g., C. hirsutus* (JP 2238885).

Suitable examples from bacteria include a laccase derivable from a strain of *Bacillus.* A laccase derived from *Coprinopsis* or *Myceliophthora* is preferred; in particular a laccase derived from *Coprinopsis cinerea,* as disclosed in WO 97/08325; or from *Myceliophthora thermophila,* as disclosed in WO 95/33836.

In a preferred embodiment of the invention, a perhydrolase is used in the method of the invention or is comprised in the medical cleaning composition of the invention.

Suitable perhydrolases are capable of catalyzing a perhydrolysis reaction that results in the production of a peracid from a carboxylic acid ester (acyl) substrate in the presence of a source of peroxygen (*e.g.*, hydrogen peroxide). While many enzymes perform this reaction at low levels, perhydrolases exhibit a high perhydrolysis:hydrolysis ratio, often greater than 1. Suitable perhydrolases may be of plant, bacterial or fungal origin. Chemically modified or protein engineered mutants are included.

Examples of useful perhydrolases include naturally occurring *Mycobacterium* perhydrolase enzymes, or variants

thereof. An exemplary enzyme is derived from *Mycobacterium smegmatis.* Such enzyme, its enzymatic properties, its structure, and variants thereof, are described in WO 2005/056782, WO 2008/063400, US 2008/145353, and US2007167344.

In a preferred embodiment of the invention the medical cleaning composition comprises at least one medical cleaning component.

The medical cleaning composition may comprise one or more wetting agents, which are surfactants, that may be anionic and/or cationic and/or non-ionic and/or semi-polar and/or zwitterionic, or a mixture thereof. In a particular embodiment, the medical cleaning composition includes a mixture of one or more nonionic surfactants and one or more anionic surfactants. The surfactant(s) is typically present at a level of from about 0% to 25% by weight, such as about 1% to about 25%, or about 3% to about 20%, or about 3% to about 10%. The surfactant(s) is chosen based on the desired cleaning application, and includes any conventional surfactant(s) known in the art. Any surfactant known in the art for use in cleaning compositions may be utilized.

When included therein the medical cleaning composition will usually contain from about 0% to about 5% by weight, such as from about 0% to about 4%, including from about 0% to about 3%, or from about 0% to about 2% of an anionic surfactant. Non-limiting examples of anionic surfactants include sulfates and sulfonates, in particular, linear alkylbenzenesulfonates (LAS), isomers of LAS, branched alkylbenzenesulfonates (BABS), phenylalkanesulfonates, alpha-olefinsulfonates (AOS), olefin sulfonates, alkene sulfonates, alkane-2,3-diylbis(sulfates), hydroxyalkanesulfonates and disulfonates, alkyl sulfates (AS) such as sodium dodecyl sulfate (SDS), fatty alcohol sulfates (FAS), primary alcohol sulfates (PAS), alcohol ethersulfates (AES or AEOS or FES, also known as alcohol ethoxysulfates or fatty alcohol ether sulfates), secondary alkanesulfonates (SAS), paraffin sulfonates (PS), ester sulfonates, sulfonated fatty acid glycerol esters, alpha-sulfo fatty acid methyl esters (alpha-SFMe or SES) including methyl ester sulfonate (MES), alkyl- or alkenylsuccinic acid, dodecenyl/tetradecenyl succinic acid (DTSA), fatty acid derivatives of amino acids, diesters and monoesters of sulfo-succinic acid or soap, and combinations thereof.

When included therein the medical cleaning composition will usually contain from about 0.1% to about 10% by weight of a cationic surfactant. Non-limiting examples of cationic surfactants include alklydimethylethanolamine quat (ADMEAQ), cetyltrimethylammonium bromide (CTAB), dimethyldistearylammonium chloride (DSDMAC), and alkylbenzyldimethylammonium, alkyl quaternary ammonium compounds, alkoxylated quaternary ammonium (AQA) compounds, and combinations thereof.

When included therein the medical cleaning composition will usually contain from about 5% to about 25% by weight of a non-ionic surfactant, for example from about 5% to about 20%, in particular from about 5% to about 15%, from about 5% to about 10%. Non-limiting examples of non-ionic surfactants include alcohol ethoxylates (AE or AEO), alcohol propoxylates, propoxylated fatty alcohols (PFA), alkoxylated fatty acid alkyl esters, such as ethoxylated and/or propoxylated fatty acid alkyl esters, alkylphenol ethoxylates (APE), nonylphenol ethoxylates (NPE), alkylpolyglycosides (APG), alkoxylated amines, fatty acid monoethanolamides (FAM), fatty acid diethanolamides (FADA), ethoxylated fatty acid monoethanolamides (EFAM), propoxylated fatty acid monoethanolamides (PFAM), polyhydroxy alkyl fatty acid amides, or N-acyl *N*-alkyl derivatives of glucosamine (glucamides, GA, or fatty acid glucamide, FAGA), as well as products available under the trade names SPAN and TWEEN, and combinations thereof.

When included therein the medical cleaning composition will usually contain from about 0.1% to about 20% by weight of a semipolar surfactant. Non-limiting examples of semipolar surfactants include amine oxides (AO) such as alkyldimethylamineoxide, *N*-(coco alkyl)-*N,N*-dimethylamine oxide and *N*-(tallow-alkyl)-*N,N*-bis(2-hydroxyethyl)amine oxide, fatty acid alkanolamides and ethoxylated fatty acid alkanolamides, and combinations thereof.

When included therein the medical cleaning composition will usually contain from about 0.1% to about 10% by weight of a zwitterionic surfactant. Non-limiting examples of zwitterionic surfactants include betaine, alkyldimethylbetaine, sulfobetaine, and combinations thereof.

In one aspect of the invention, the wash liquor and/or the medical cleaning composition comprises a hydrotrope. A hydrotrope is a compound that solubilises hydrophobic compounds in aqueous solutions (or oppositely, polar substances in a non-polar environment). Typically, hydrotropes have both hydrophilic and a hydrophobic character (so-called amphiphilic properties as known from surfactants); however, the molecular structure of hydrotropes generally do not favor spontaneous self-aggregation, see for example review by Hodgdon and Kaler (2007), Current Opinion in Colloid & Interface Science 12: 121-128. Hydrotropes do not display a critical concentration above which self-aggregation occurs as found for surfactants and lipids forming miceller, lamellar or other well defined meso-phases. Instead, many hydrotropes show a continuous-type aggregation process where the sizes of aggregates grow as concentration increases. However, many hydrotropes alter the phase behavior, stability, and colloidal properties of systems containing substances of polar and non-polar character, including mixtures of water, oil, surfactants, and polymers. Hydrotropes are classically used across industries from pharma, personal care, food, to technical applications. Use of hydrotropes in cleaning compositions allow for example more concentrated formulations of surfactants (as in the process of compacting liquid cleaning compositions by removing water) without inducing undesired phenomena such as phase separation or high viscosity.

The medical cleaning composition may contain 0-5% by weight, such as about 0.5 to about 5%, or about 3% to about 5%, of a hydrotrope. Any hydrotrope known in the art for use in cleaning compositions may be utilized. Non-limiting examples of hydrotropes include sodium benzene sulfonate, sodium p-toluene sulfonate (STS), sodium xylene sulfonate (SXS), sodium cumene sulfonate (SCS), sodium cymene sulfonate, amine oxides, alcohols and polyglycolethers, sodium hydroxynaphthoate, sodium hydroxynaphthalene sulfonate, sodium ethylhexyl sulfate, and combinations thereof.

In one aspect of the invention, the wash liquor and/or the medical cleaning composition comprises a detergent builder or co-builder.

The medical cleaning composition may contain about 0-65% by weight, such as about 5% to about 50% of a detergent builder or co-builder, or a mixture thereof. The builder and/or co-builder may particularly be a chelating agent that forms water-soluble complexes with Ca and Mg ions. Any builder and/or co-builder known in the art for use in laundry detergents may be utilized. Non-limiting examples of builders include citrates, zeolites, diphosphates (pyrophosphates), triphosphates such as sodium triphosphate (STP or STPP), carbonates such as sodium carbonate, soluble silicates such as sodium metasilicate, layered silicates (e.g., SKS-6 from Hoechst), ethanolamines such as 2-aminoethan-1-ol (MEA), diethanolamine (DEA, also known as iminodiethanol), triethanolamine (TEA, also known as 2,2',2"-nitrilotriethanol), and carboxymethyl inulin (CMI), and combinations thereof.

The medical cleaning composition may also contain 0-50% by weight, such as about 0.5% to about 10%, of a detergent co-builder, or a mixture thereof. The medical cleaning composition may include include a co-builder alone, or in combination with a builder, for example a citrate builder. Non-limiting examples of co-builders include homopolymers of polyacrylates or copolymers thereof, such as poly(acrylic acid) (PAA) or copoly(acrylic acid/maleic acid) (PAA/PMA). Further non-limiting examples include citrate, chelators such as aminocarboxylates, aminopolycarboxylates and phosphonates, and alkyl- or alkenylsuccinic acid. Additional specific examples include 2,2',2"-nitrilotriacetic acid (NTA), ethylenediaminetetraacetic acid (EDTA), diethylenetriaminepentaacetic acid (DTPA), iminodisuccinic acid (IDS), ethylenediamine-*N,N'*-disuccinic acid (EDDS), methylglycinediacetic acid (MGDA), glutamic acid-N,N-diacetic acid (GLDA), 1-hydroxyethane-1,1-diphosphonic acid (HEDP), ethylenediaminetetra(methylenephosphonic acid) (EDTMPA), diethylenetriaminepentakis(methylenephosphonic acid) (DTMPA or DTPMPA), *N*-(2-hydroxyethyl)iminodiacetic acid (EDG), aspartic acid-*N*-monoacetic acid (ASMA), aspartic acid-*N*,*N*-diacetic acid (ASDA), aspartic acid-*N*-monopropionic acid (ASMP), iminodisuccinic acid (IDA), *N*-(2-sulfomethyl)-aspartic acid (SMAS), *N*-(2-sulfoethyl)-aspartic acid (SEAS), *N*-(2-sulfomethyl)-glutamic acid (SMGL), *N*-(2-sulfoethyl)-glutamic acid (SEGL), *N*-methyliminodiacetic acid (MIDA), α-alanine-*N*, *N*-diacetic acid (α-ALDA), serine-*N*, *N*-diacetic acid (SEDA), isoserine-*N*, *N*-diacetic acid (ISDA), phenylalanine-*N*, *N*-diacetic acid (PHDA), anthranilic acid-*N*, *N*-diacetic acid (ANDA), sulfanilic acid-*N*, *N*-diacetic acid (SLDA), taurine-*N*, *N*-diacetic acid (TUDA) and sulfomethyl-*N*, *N*-diacetic acid (SMDA), *N*-(2-hydroxyethyl)-ethylidenediamine-*N*, *N'*, *N'*-triacetate (HEDTA), diethanolglycine (DEG), diethylenetriamine penta(methylenephosphonic acid) (DTPMP), aminotris(methylenephosphonic acid) (ATMP), and combinations and salts thereof. Further exemplary builders and/or co-builders are described in, *e.g.*, WO 09/102854, US 5977053.

In one aspect of the invention, the wash liquor and/or the medical cleaning composition comprises a polymer.

The medical cleaning composition may contain 0-10% by weight, such as 0.5-5%, 2-5%, 0.5-2% or 0.2-1% of a polymer. Any polymer known in the art for use in cleaning compositions may be utilized. The polymer may function as a co-builder as mentioned above, or may provide soil release, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(vinylpyrrolidone) (PVP), poly(ethyleneglycol) or poly(ethylene oxide) (PEG), ethoxylated poly(ethyleneimine), carboxymethyl inulin (CMI), and polycarboxylates such as PAA, PAA/PMA, poly-aspartic acid, and lauryl methacrylate/acrylic acid copolymers , hydrophobically modified CMC (HM-CMC) and silicones, copolymers of terephthalic acid and oligomeric glycols, copolymers of poly(ethylene terephthalate) and poly(oxyethene terephthalate) (PET-POET), PVP, poly(vinylimidazole) (PVI), poly(vinylpyridine-N-oxide) (PVPO or PVPNO) and polyvinylpyrrolidone-vinylimidazole (PVPVI). Further exemplary polymers include sulfonated polycarboxylates, polyethylene oxide and polypropylene oxide (PEO-PPO) and diquaternium ethoxy sulfate. Other exemplary polymers are disclosed in, *e.g.*, WO 2006/130575 and US 5,955,415. Salts of the above-mentioned polymers are also contemplated.

In one aspect of the invention, the wash liquor and/or the medical cleaning composition comprises a bleaching system.

Due to the incompatibility of the components there are still only few examples of liquid cleaning compositions combining bleach and enzymes (*e.g.*, US 5,275,753 or WO 99/00478). The medical cleaning composition may contain 0-50% of a bleaching system. Any bleaching system known in the art for use in laundry cleaning compositions may be utilized. Suitable bleaching system components include bleaching catalysts, photobleaches, bleach activators, sources of hydrogen peroxide such as sodium percarbonate and sodium perborates, preformed peracids and mixtures thereof. Suitable preformed peracids include, but are not limited to, peroxycarboxylic acids and salts, percarbonic acids and salts, perimidic acids and salts, peroxymonosulfuric acids and salts, for example, Oxone (R), and mixtures thereof. Non-limiting examples of bleaching systems include peroxide-based bleaching systems, which may comprise, for example, an inorganic salt, including alkali metal salts such as sodium salts of perborate (usually mono- or tetra-hydrate), percarbonate, persulfate, perphosphate, persilicate salts, in combination with a peracid-forming bleach activator. The term bleach activator is meant herein as a compound which reacts with peroxygen bleach like hydrogen peroxide to form a peracid. The peracid thus formed constitutes the activated bleach. Suitable bleach activators to be used herein include those belonging to the class of esters amides, imides or anhydrides. Suitable examples are tetracetylethylene diamine (TAED), sodium 4-[(3,5,5-trimethylhexanoyl)oxy]benzene sulfonate (ISONOBS), diperoxy dodecanoic acid, 4-(dodecanoyloxy)benzenesulfonate (LOBS), 4-(decanoyloxy)benzenesulfonate, 4-(decanoyloxy)benzoate (DOBS), 4-(nonanoyloxy)-benzenesulfonate (NOBS), and/or those disclosed in WO 98/17767. A particular family of bleach activators of interest was disclosed in EP624154 and particulary preferred in that family is acetyl triethyl citrate (ATC). ATC or a short chain triglyceride like triacetin has the advantage that it is environmentally friendly as it eventually degrades into citric acid and alcohol. Furthermore, acetyl triethyl citrate and triacetin has a good hydrolytical stability in the product upon storage and it is an efficient bleach activator. Alternatively, the bleaching system may comprise peroxyacids of, for example, the amide, imide, or sulfone type. The bleaching system may also comprise peracids such as 6-(phthalimido)peroxyhexanoic acid (PAP). The bleaching system may also include a bleach catalyst. In some embodiments the bleach component may be an organic catalyst selected from the group consisting of organic catalysts having the following formulae:

(i)

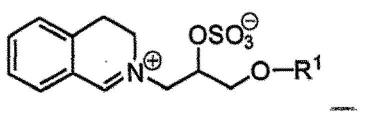

(ii)

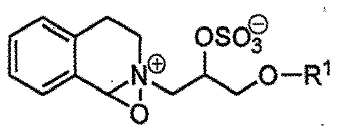

and mixtures thereof; wherein each $R^1$ is independently a branched alkyl group containing from 9 to 24 carbons or linear alkyl group containing from 11 to 24 carbons, preferably each $R^1$ is independently a branched alkyl group containing from 9 to 18 carbons or linear alkyl group containing from 11 to 18 carbons, more preferably each $R^1$ is independently selected from the group consisting of 2-propylheptyl, 2-butyloctyl, 2-pentylnonyl, 2-hexyldecyl, n-dodecyl, n-tetradecyl, n-hexadecyl, n-octadecyl, iso-nonyl, iso-decyl, iso-tridecyl and iso-pentadecyl. Other exemplary bleaching systems are described, *e.g.*, in WO 2007/087258, WO 2007/087244, WO 2007/087259 and WO 2007/087242. Suitable photobleaches may for example be sulfonated zinc phthalocyanine.

In one aspect of the invention, the wash liquor and/or the medical cleaning composition comprises at least one polymer.

The detergent may contain 0.005-10% by weight, such as 0.5-5%, 2-5%, 0.5-2% or 0.2-1% of a polymer. Any polymer known in the art for use in detergents may be utilized. The polymer may function as a co-builder as mentioned above, or may provide antiredeposition, fiber protection, soil release, dye transfer inhibition, grease cleaning and/or anti-foaming properties. Some polymers may have more than one of the above-mentioned properties and/or more than one of the below-mentioned motifs. Exemplary polymers include (carboxymethyl)cellulose (CMC), poly(vinyl alcohol) (PVA), poly(ethyleneglycol) or poly(ethylene oxide) (PEG or PEO), ethoxylated poly(ethyleneimine), (carboxymethyl)inulin (CMI), carboxylate polymers and polycarboxylates such as polyacrylates, maleic/acrylic acid copolymers, acrylate/styrene copolymers, poly(aspartic) acid, and lauryl methacrylate/acrylic acid copolymers, hydrophobically modified CMC (HM-CMC), silicones, copolymers of terephthalic acid and oligomeric glycols, copolymers of poly(ethylene terephthalate) and poly(oxyethene terephthalate) (PET-POET), poly(vinylpyrrolidone) (PVP), poly(vinylimidazole) (PVI), poly(vinylpyridine-*N*-oxide) (PVPO or PVPNO) and copoly(vinylimidazole/vinylpyrrolidone) (PVPVI). Suitable examples include PVP-K15, PVP-K30, ChromaBond S-400, ChromaBond S- 403E and Chromabond S-100 from Ashland Aqualon, and Sokalan® HP 165, Sokalan® HP 50 (Dispersing agent), Sokalan® HP 53 (Dispersing agent), Sokalan® HP 59 (Dispersing agent), Sokalan® HP 56 (dye transfer inhibitor), Sokalan® HP 66 K (dye transfer inhibitor) from BASF. Further exemplary polymers include sulfonated polycarboxylates, polyethylene oxide and polypropylene oxide (PEO-PPO) and diquaternium ethoxy sulfate. Particularly preferred polymer is ethoxylated homopolymer Sokalan® HP 20 from BASF, which

helps to prevent redeposition of soil in the wash liquor. Further exemplary polymers include sulfonated polycarboxylates, ethylene oxide-propylene oxide copolymers (PEO-PPO), copolymers of PEG with and vinyl acetate, and diquaternium ethoxy sulfate or quaternized sulfated ethoxylated hexamethylenediamine. Other exemplary polymers are disclosed in, e.g., WO 2006/130575. Salts of the above-mentioned polymers are also contemplated.

The medical cleaning components can be separated physically from each other by compartments in water dissolvable pouches. Thereby negative storage interaction between components can be avoided. Different dissolution profiles of each of the compartments can also give rise to delayed dissolution of selected components in the wash solution.

The invention is further summarized in the following embodiments of the invention.

1. A method for cleaning a medical device comprising the steps of:

a) Providing a wash liquor having a pH in the range of 6.0-10.0, comprising a polypeptide having DNase activity and optionally further cleaning components,
b) Subjecting at least part of the medical device to the wash liquor of step a),
c) Optionally, subjecting the medical device to rinsing,
d) Optionally, subjecting the medical device to drying, and
e) Optionally, subjecting the medical device to sterile packaging.

2. The method according to embodiment 1, wherein at least a part of the medical device comprises organic soil.
3. The method according to any of the preceding embodiments, wherein the organic soil comprises one or more microorganisms.
4. The method according to any of the preceding embodiments, wherein the microorganisms is selected from the group consisting of *Acinetobacter sp., Aeromicrobium sp., Aspergillus versicolor, Brevundimonas sp., Enterobacter cloacae, Enterococcus faecalis, Escherichia coli, Klebsiella pneumoniae, Microbacterium sp., Micrococcus luteus, Pseudomonas sp., Staphylococcus aureus, Staphylococcus epidermidis, Stenotrophomonas sp., Bacillus sp.,* and *Pseudomonas aeruginosa.*
5. The method according to embodiment 4, wherein the microorganisms comprises *Pseudomonas aeruginosa.*
6. The method according to any of the preceding embodiments, wherein the wash liquor comprising a polypeptide having DNase activity releases and/or removes at least part of the organic soil present on the part of the medical device subjected to the wash liquor.
7. The method according to any of the preceding embodiments, wherein the medical device is disinfected before being subjected to the wash liquor.
8. The method according to any of the preceding embodiments, wherein substantially no live microorganisms are comprised in the organic soil.
9. The method according to embodiment 8, wherein at least part of the medical device is subjected to a liquid solution comprising peracetic acid having a pH of 3, whereafter no live microorganisms are comprised in the organic soil.
10. The method according to any of the preceding embodiments, wherein at least 95% of the microorganisms comprised in the organic soil are dead.
11. The method according to any of the preceding embodiments, wherein the disinfection is performed by subjecting at least part of the medical device to a chemical or a biological disinfection composition or to radiation.
12. The method according to embodiment 11, wherein the disinfection is performed by subjecting at least part of the medical device to antimicrobial agents, antibiotics, fungicides, oxidizing agents, hydrogen peroxide, chlorine, acids, peroxy acids, peracetic acid, potassium permanganate, ultra-violet radiation (UV), ozon.
13. The method according to any of the preceding embodiments, wherein the pH of the wash liquor is in the range of 6.5-9.5, preferably in the range of 7.0-9.0, more preferably in the range of 7.5-8.5, such as a pH around 8.
14. The method according to any of the preceding embodiments, wherein the wash liquor comprises at least 0.001 ppm of the polypeptide having DNase activity, preferably at least 0.005 ppm of the polypeptide having DNase activity, more preferably at least 0.01 ppm of the polypeptide having DNase activity, even more preferably at least 0.05 ppm of the polypeptide having DNase activity or most preferably at least 1 ppm of the polypeptide having DNase activity.
15. The method according to any of the preceding embodiments, wherein the wash liquor comprises at most 100 ppm of the polypeptide having DNase activity, preferably at most 50 ppm of the polypeptide having DNase activity, more preferably at most 30 ppm of the polypeptide having DNase activity, even more preferably at most 20 of the polypeptide having DNase activity or most preferably at most 10 ppm of the polypeptide having DNase activity.
16. The method according to any of the preceding embodiments, wherein the wash liquor comprises in the range of 0.001-100 ppm of the polypeptide having DNase activity, preferably in the range of 0.005-50 ppm of the polypeptide having DNase activity, more preferably in the range of 0.01-30 ppm of the polypeptide having DNase activity, even more preferably in the range of 0.05-20 ppm of the polypeptide having DNase activity or most preferably in the range of 1-10 ppm more of the polypeptide having DNase activity.

17. The method according to any of the preceding embodiments, wherein the wash liquor further comprises one or more cleaning composition components, preferably selected from surfactants, builders, bleach components, polymers, dispersing agents and additional enzymes.
18. The method according to any of the preceding embodiments, wherein the wash liquor further comprises one or more enzymes selected from the group consisting of proteases, amylases, lipases, cutinases, cellulases, endoglucanases, xyloglucanases, pectinases, pectin lyases, xanthanases, peroxidaes, haloperoxygenases, catalases, galactanase, mannanases, or any mixture thereof.
19. The method according to any of the preceding embodiments, wherein at least part of the medical device is subjected to the wash liquor by spraying, dipping or immersing.
20. The method according to any of the preceding embodiments, wherein the medical device is subjected to the wash liquor by immersing at least part of the medical device into the wash liquor.
21. The method according to embodiment 20, wherein at least part of the medical device is soaked in the wash liquor for a time sufficient to reduce and/or remove soil on the instrument, preferably for at least 1 minute.
22. The method according to any of the preceding embodiments, wherein the medical device is subjected to rinsing after being subjected to the wash liquor.
23. The method according to any of the preceding embodiments, wherein the medical device is subjected to drying after being subjected to the wash liquor.
24. The method according to any of the preceding embodiments, wherein the medical device is subjected to sterile packaging after being subjected to the wash liquor.
25. The method according to any of the preceding embodiments, wherein the wash liquor comprising a polypeptide having DNase activity releases and/or removes at least 50% of the organic soil present on the part of the medical device subjected to the wash liquor as measured in Example 2, preferably at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.
26. The method according to any of the preceding embodiments, wherein the medical device is an indwelling device.
27. The method according to any of the preceding embodiments, wherein the polypeptide having DNase activity is of bacterial or fungal origin.
28. The method according to any of the preceding embodiments wherein the polypeptide having DNase activity is obtained from *Bacillus* or *Aspergillus.*
29. The method according to embodiment 27, wherein the polypeptide having DNase activity is obtained from *Bacillus cibi* or *Aspergillus oryzae.*
30. The method according to any of the preceding embodiments, wherein the polypeptide having DNase activity is selected from the group consisting of:

a) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 1;
b) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 2;
c) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 3;
d) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 4;
e) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 5;
f) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 6;
g) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 7;
h) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%,

at least 98%, at least 99%, or 100% sequence identity to the polypeptide shown in SEQ ID NO: 8;
i) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 9 and
j) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 10.

31. A medical cleaning composition comprising a polypeptide having DNase activity, wherein the composition has a pH in the range of 6.0-10.0.
32. A medical cleaning composition according to embodiment 31, wherein the polypeptide is selected from the group consisting of:

a) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 1;
b) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 2;
c) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 3;
d) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 4;
e) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 5;
f) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 6;
g) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 7;
h) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 8;
i) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 9 and
j) a polypeptide having at least 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 10.

33. The medical cleaning composition according to embodiment 32, wherein the composition further comprises one or more cleaning composition components, preferably selected from surfactants, builders, bleach components, polymers, dispersing agents and additional enzymes.
34. The medical cleaning composition according any of embodiments 31-33, wherein the medical cleaning composition further comprises one or more enzymes selected from the group consisting of proteases, amylases, lipases, cutinases, cellulases, endoglucanases, xyloglucanases, pectinases, pectin lyases, xanthanases, peroxidaes, haloperoxygenases, catalases, galactanase, mannanases, or any mixture thereof.
35. The medical cleaning composition according to any of embodiments 31-34, wherein the composition comprises a protease and/or a mannanase, wherein the protease is:

a) a polypeptide having 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 11;
b) a polypeptide having 60%, e.g., at least 65%, at least 70%, at least 75%, at least 80%, at least 85%, at least

90%, at least 91%, at least 92%, at least 93%, at least 94%, at least 95%, at least 96%, at least 97%, at least 98%, at least 99%, or 100% sequence identity to the polypeptide shown in SEQ ID NO: 12,
and wherein the mannanase is:
c) a polypeptide having 60%, e.g., at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in sequence identity to the polypeptide shown in SEQ ID NO: 13.

36. The medical cleaning composition according any of embodiments 31-35 comprising 5-25% of a non-ionic surfactant and 0-5% of an anionic surfactant.
37. The medical cleaning composition according embodiment 36, wherein the composition comprises 5-20% of a non-ionic surfactant, preferably 5-15% or more preferably 5-10% of a non-ionic surfactant.
38. The medical cleaning composition according embodiments 36-37, wherein the composition comprises 0-4% of a anionic surfactant, preferably 0-3%, or more preferably 0-2% of a anionic surfactant.
39. The medical cleaning composition according to any of embodiments 31-38, wherein the composition is in the form of a bar, a homogenous tablet, a tablet having two or more layers, a pouch having one or more compartments, a regular or compact powder, a granule, a paste, a gel, a regular liquid, a compact liquid or concentrated liquid.
40. Use of a polypeptide having DNase activity for releasing and/or removing or organic soil from a medical device.
41. Use according to embodiment 40, wherein the polypeptide having DNase activity is used for coating at least part of a medical device.
42. Use according to any of embodiments 40-41, wherein the medical device is an indwelling device.
43. Use according to any of embodiments 40-42, wherein the medical device is selected from the group consisting of a catheter such as a central venous catheter, intravascular catheter, urinary catheter, Hickman catheter, peritoneal dialysis catheter, endrotracheal catheter, or wherein the device is a mechanical heart valve, a cardiac pacemaker, an arteriovenous shunt, a scleral buckle, a prosthetic joint, a tympanostomy tube, a tracheostomy tube, a voice prosthetic, a penile prosthetic, an artificial urinary sphincter, a synthetic pubovaginal sling, a surgical suture, a bone anchor, a bone screw, an intraocular lens, a contact lens, an intrauterine device, an aortofemoral graft, a vascular graft, a needle, a Luer-Lok connector, a needleless connector, surgical instrument, diagnostic instruments, trays, pans, holders, racks, forceps, scissors, shears, saws (e.g. bone saws and their blades), hemostats, knives, chisels, rongeurs, files, nippers, drills, drill bits, rasps, burrs, spreaders, breakers, elevators, clamps, needle holders, carriers, clips, hooks, gouges, curettes, retractors, straightener, punches, extractors, scoops, keratomes, spatulas, expressors, trocars, dilators, cages, glassware, tubing, catheters, cannulas, plugs, stents, endoscopes, arthoscopes and related equipment.
44. Medical device coated with a composition comprising a polypeptide having DNase activity.

**CLEANING COMPOSITIONS**

Any of the medical cleaning compositions described below, may include one or more polypeptides having DNase activity and any number of additional enzymes. In general, the enzyme(s) should be compatible with the selected cleaning composition, (e.g., with respect to pH-optimum, compatibility with other enzymatic and non-enzymatic ingredients, and the like), and the enzyme(s) should be present in effective amounts. The medical cleaning composition are commercially available.

Dr. Weigert Neodisher Multizym (Dr. Weigert)

5-15% non-ionic surfactants, less than 5% anionic surfactants, enzymes, preservation agents: 2-Octyl-2H-isothiazol-3-one and methylisothiazolinone.

Aniosyme Synergy 5 (Anios Laboratoires)

2.5-10% N,N-dimethyldecylamine N-oxide, less than 2.5% sodium methane sulphonate, less than 2.5% dicethyldioctylammonium chloride and less than 2.5% of a 3:1 mixture of 5-chlor-2-methyl-2H-isothiazol-3-on and 2-methyl-2H-isothiazol-3-on.

Metrex Metrizyme (Metrex)

10-30% propylene glycol, 10-20% non-ionic surfactant, 5-10% fragrance oil, 1.1-1% proteinase substilisin and a 30-60% of a mixture of water and non-harzadous ingredients.

Steris Prolystica 2X Enzymatic Presoak and Cleaner (Steris)

1-5% alcohols, C9-11, ethoxylated surfactant,

1-5% glycerin, 1-5% citric acid, 1-2% N,N-dimethyloctadecylamine oxide and 0.1-1% substilisins (proteolytic enzyme).

**ENZYME ASSAYS**

Assay I: testing of DNase activity

DNase activity was determined on DNase Test Agar with Methyl Green (BD, Franklin Lakes, NJ, USA), which was prepared according to the manual from supplier. Briefly, 21 g of agar was dissolved in 500 ml water and then autoclaved for 15 min at 121°C. Autoclaved agar was temperated to 48°C in water bath, and 20 ml of agar was poured into petridishes with and allowed to solidify by incubation o/n (over night) at room temperature. On solidified agar plates, 5 μl of enzyme solutions are added, and DNase activity are observed as colorless zones around the spotted enzyme solutions.

**EXAMPLES**

**Example 1**

**Removal effect of DNase and other enzymes in model detergent on *Pseudomonas aeruginosa* dead biofilm**

Two different biofilms were used in the example. One produced by *Pseudomonas aeruginosa* PA14 (DSM19882) and one produced by *P. aeruginosa* PA01 (DSM22644). The bacteria were re-streaked on tryptic soy agar plates and incubated for 3 days at 30°C. After 3 days of incubation, tryptic soy broth (TSB) was inoculated with one colony of *P. aeruginosa* PA14 (DSM 19882) or one colony of *P. aeruginosa* PA01 (DSM22644). The inoculated TSB tubes were all incubated overnight at 30°C. The overnight cultures were diluted in TSB to a specific optical density (OD). To each well in Thermo Scientific™ Nunc™ MicroWell™ 96-Well Microplates (sterile, non-treated) 150 μl of overnight culture was added. The plates were incubated at 30°C for 4 hours.

After 4 hours of incubation the microtiter plates (MTP) containing biofilm were removed from the incubator and emptied for media using VacusafeTM Vacuum Aspiration System (INTEGRA Biosciences). Each well was rinsed twice with 200 μl 0.9% NaCl solution. After rinse, 200 μl of 1000 ppm peracetic acid (PAA) (pH 3) was added to each well for complete kill of the bacteria. The MTPs were incubated with PAA for 10 minutes at 30°C. After PAA treatment each well was rinsed twice with 200 μl 0.9% NaCl solution.

The complete kill of microorganism was verified by PAA treatment of a biofilm grown under the same conditions as described above. After treatment with 200 μL of 1000 ppm PAA for 10 minutes and rinsing with 2 x 200 μL 0.9% NaCl in each well, 200 μL of fresh TSB was added to each well. The MTP was placed at 30°C for 4 days, whereafter each well was checked for regrowth by measuring the OD at 595 nm. No regrowth was observed in any of the wells.

A model detergent liquor was prepared by dissolving 5 g/l model detergent containing 5% MPG (mono propylene glycol), 5% Pluronic PE 4300 (PO/EO block polymer; 70%/30%, approx. 1750 g/mol), 2% Plurafac LF 305 (fatty alcohol alkoxylate; C6-10 + EO/PO), 1% MGDA (methyl glycine diacetic acid, 1% TEA (triethanolamine), pH-adjusted to 8.7 with phosphoric acid as water to 100% (all percentages are w/w (weight volume)) in water with hardness 5°dH.

To each well in the MTPs, 200 μL of treatment solution (model detergent liquor ± enzymes) was added. Each enzyme was applied at a final concentration of 5 μg/mL Each treatment was applied to eight wells.

After addition of model detergent liquor +/- enzyme, the MTPs were incubated static for 60 minutes at 30°C. After 60 minutes of incubation, the treatment liquor was removed using the vacuum system. Each well was rinsed twice with 200 μl 0.9% NaCl solution, and 200 μl of 0.095% crystal violet solution was added to each well. The MTPs were incubated for 15 minutes at room temperature. The crystal violet solution was removed using the vacuum system, and each well was rinsed twice with 200 μl 0.9% NaCl solution. 150 μl of 30% acetic acid was added to each well. The MTPs were incubated for 10 minutes at room temperature, whereafter the absorbance at 595 nm was measured using a spectrophotometer (SpectraMax M3, Molecular Devices). The MTPs were shaked for 10 seconds before absorbance measurements were performed.

The enzymes tested in example 1 are listed in table 1 below.

Table 1:

| Protease |
|---|

(continued)

| |
|---|
| Mannanase |
| DNase |

The % remaining dead biofilm after enzymatic treatment was calculated as $ABS_{595(\text{biofilm treated with model detergent+enzyme})}/ABS_{595(\text{biofilm treated with model detergent})}$ x 100%.

The results are displayed in table 2 and figure 1.

Table 2: %remaining dead biofilm after treatment with DNase, protease and/or mannanase in 0.5% model detergent wash liquor

| | *P. aeruginosa* PA14 (DSM19882) | *P. aeruginosa* PA01 (DSM22644) |
|---|---|---|
| 0.5% Model detergent | 100 | 100 |
| 0.5% Model detergent + 5 μg/mL protease | 74 | 108 |
| 0.5% Model detergent + 5 μg/mL Mannanase | 87 | 100 |
| 0.5% Model detergent + 5 μg/mL DNase | 11 | 9 |
| 0.5% Model detergent + 5 μg/mL protease + 5 μg/mL Mannanase | 72 | 102 |
| 0.5% Model detergent + 5 μg/mL protease + 5 μg/mL + DNase | 8 | 7 |
| 0.5% Model detergent + 5 μg/mL Mannanase + 5 μg/mL DNase | 11 | 12 |
| 0.5% Model detergent + 5 μg/mL protease + 5 μg/mL Mannanase + 5 μg/mL DNase | 8 | 5 |

**Example 2**

**Dosage response removal effect of DNase in model detergent on *Pseudomonas aeruginosa* dead biofilm**

4-hour old and 24-hour old *P. aeruginosa* PA14 (DSM19882) and *P. aeruginosa* PA01 (DSM22644) dead biofilms were produced as described in example 1, except that both aeruginosa strains were grown for 4-hours and 24-hours before kill using 1000 ppm PAA. After kill using PAA and rinsing using 0.9% NaCl, 200 μL of treatment solutions (model detergent ± enzymes) were added to each well. DNase was applied in model detergent liquor (as described in example 1) in dilutions from 0 - 5 μg/mL. Each treatment was applied to eight wells. After addition of model detergent +/- enzyme, the microtiter plates were incubated static for 60 minutes at 30°C. After 60 minutes of incubation, the remaining amount of dead biofilm was quantified, as described in example 1, by staining with crystal violet and resolubilizing in 30% acetic acid. Finally, the absorbance was measured at 595 nm. The %remaining dead biofilm after enzymatic treatment was calculated as $ABS_{595(\text{biofilm treated with model detergent+enzyme})}/ABS_{595(\text{biofilm treated with model detergent})}$ X 100%. The results are displayed in table 3 and in Figure 2 and Figure 3.

Table 3: %remaining dead biofilm after treatment with increasing dosage of DNase in 0.5% model detergent

| | ***P. aeruginosa* PA14 (DSM 19882)** | | ***P. aeruginosa* PA01 (DSM22644)** | |
|---|---|---|---|---|
| **Dosage of DNase (mg/L)** | **4-hour old biofilm (dead)** | **24-hour old biofilm (dead)** | **4-hour old biofilm (dead)** | **24-hour old biofilm (dead)** |
| **0** | 100 | 100 | 100 | 100 |
| **0.01** | 52 | 72 | 74 | 36 |
| **0.025** | 42 | 68 | 68 | 37 |

(continued)

| | *P. aeruginosa* PA14 (DSM 19882) | | *P. aeruginosa* PA01 (DSM22644) | |
|---|---|---|---|---|
| **Dosage of DNase (mg/L)** | **4-hour old biofilm (dead)** | **24-hour old biofilm (dead)** | **4-hour old biofilm (dead)** | **24-hour old biofilm (dead)** |
| **0.05** | 14 | 68 | 45 | 32 |
| **0.1** | 14 | 67 | 49 | 29 |
| **0.25** | 6 | 55 | 23 | 18 |
| **0.5** | 5 | 45 | 12 | 14 |
| **1** | 6 | 37 | 10 | 11 |
| **2.5** | 6 | 25 | 8 | 9 |
| **5** | 5 | 26 | 8 | 8 |

**Example 3**

**Effect of time and dosage response of DNase in commercial detergents on *Pseudomonas aeruginosa* dead biofilm**

In the current example, the dead biofilm removal effect of DNase was tested in four different commercially available endoscopic reprocessing detergents. Two detergents from EU, and two detergents from US. Dead biofilm from *P. aeruginosa* PA14 was produced for 4-hours as described in Example 1. DNase was applied in each detergent to the dead biofilm in dosages from 0-5 μg/mL for 5 minutes, 15 minutes, 30 minutes and 60 minutes. The detergents were applied in the mid of the recommended dosage range. Unwashed control wells were included in the examples, where 200 μl 0.9% NaCl solution was added to the unwashed wells during treatment time. The remaining dead biofilm was quantified, as described in example 1, by staining with crystal violet and resolubilizing in 30% acetic acid. The absorbance was measured at 595 nm, where a high amount of remaining biofilm result in a high absorbance value. The results are displayed in tables 4-7 and in figures 4-7 as absorbance values.

Table 4: $ABS_{595nm}$ of crystal violet stained remaining dead biofilm after treatment with increasing dosage of DNase in EU detergent 1, Dr. Weigert Neodisher Multizym, for 5, 15, 30 and 60 minutes

| **Treatment time** | **5 min** | **15 min** | **30 min** | **60 min** |
|---|---|---|---|---|
| **Unwashed** | 0,592 | 0,526 | 0,556 | 0,676 |
| **EU detergent 1** | 0,243 | 0,259 | 0,207 | 0,209 |
| **EU detergent 1 + 0.025 ppm DNase** | 0,146 | 0,125 | 0,089 | 0,072 |
| **EU detergent 1 + 0.05 ppm DNase** | 0,108 | 0,097 | 0,069 | 0,055 |
| **EU detergent 1 + 0.1 ppm DNase** | 0,107 | 0,084 | 0,078 | 0,048 |
| **EU detergent 1 + 0.25 ppm DNase** | 0,067 | 0,071 | 0,071 | 0,051 |
| **EU detergent 1 + 0.5 ppm DNase** | 0,073 | 0,067 | 0,049 | 0,037 |
| **EU detergent 1 + 1 ppm DNase** | 0,068 | 0,060 | 0,040 | 0,026 |
| **EU detergent 1 + 2.5 ppm DNase** | 0,053 | 0,033 | 0,036 | 0,034 |
| **EU detergent 1 + 5 ppm DNase** | 0,048 | 0,024 | 0,039 | 0,041 |

Table 5: $ABS_{595nm}$ of crystal violet stained remaining dead biofilm after treatment with increasing dosage of DNase in EU detergent 2, Aniosyme Synergy 5, for 5, 15, 30 and 60 minutes

| Treatment time | 5 min | 15 min | 30 min | 60 min |
|---|---|---|---|---|
| Unwashed | 0,582 | 0,593 | 0,746 | 1,173 |
| EU detergent 2 | 0,316 | 0,587 | 0,402 | 0,465 |
| EU detergent 2 + 0.025 ppm DNase | 0,189 | 0,175 | 0,093 | 0,101 |
| EU detergent 2 + 0.05 ppm DNase | 0,158 | 0,123 | 0,069 | 0,075 |
| EU detergent 2 + 0.1 ppm DNase | 0,141 | 0,097 | 0,062 | 0,069 |
| EU detergent 2 + 0.25 ppm DNase | 0,125 | 0,090 | 0,055 | 0,060 |
| EU detergent 2 + 0.5 ppm DNase | 0,113 | 0,080 | 0,038 | 0,042 |
| EU detergent 2 + 1 ppm DNase | 0,094 | 0,056 | 0,024 | 0,025 |
| EU detergent 2 + 2.5 ppm DNase | 0,074 | 0,049 | 0,019 | 0,024 |
| EU detergent 2 + 5 ppm DNase | 0,042 | 0,031 | 0,014 | 0,028 |

Table 6: $ABS_{595nm}$ of crystal violet stained remaining dead biofilm after treatment with increasing dosage of DNase in US detergent 1, Steris Prolysitica 2X Enzymatic Presoak and Cleaner, for 5, 15, 30 and 60 minutes

| Treatment time | 5 min | 15 min | 30 min | 60 min |
|---|---|---|---|---|
| Unwashed | 0,276 | 0,432 | 0,389 | 0,437 |
| US detergent 1 | 0,205 | 0,223 | 0,220 | 0,269 |
| US detergent 1 + 0.025 ppm DNase | 0,171 | 0,126 | 0,079 | 0,098 |
| US detergent 1 + 0.05 ppm DNase | 0,141 | 0,096 | 0,058 | 0,070 |
| US detergent 1 + 0.1 ppm DNase | 0,115 | 0,086 | 0,064 | 0,066 |
| US detergent 1 + 0.25 ppm DNase | 0,100 | 0,063 | 0,053 | 0,057 |
| US detergent 1 + 0.5 ppm DNase | 0,087 | 0,053 | 0,040 | 0,051 |
| US detergent 1 + 1 ppm DNase | 0,069 | 0,045 | 0,036 | 0,047 |
| US detergent 1 + 2.5 ppm DNase | 0,048 | 0,030 | 0,026 | 0,044 |
| US detergent 1 + 5 ppm DNase | 0,034 | 0,027 | 0,022 | 0,051 |

Table 7: $ABS_{595nm}$ of crystal violet stained remaining dead biofilm after treatment with increasing dosage of DNase in US detergent 2, Metrex Metrizyme, for 5, 15, 30 and 60 minutes

| **Treatment time** | **5 min** | **15 min** | **30 min** | **60 min** |
|---|---|---|---|---|
| **Unwashed** | 0,613 | 0,598 | 0,599 | 0,658 |
| **US detergent 2** | 0,237 | 0,239 | 0,202 | 0,222 |
| **US detergent 2 + 0.025 ppm DNase** | 0,100 | 0,048 | 0,026 | 0,007 |
| **US detergent 2 + 0.05 ppm DNase** | 0,088 | 0,052 | 0,020 | 0,008 |
| **US detergent 2 + 0.1 ppm DNase** | 0,080 | 0,044 | 0,015 | 0,007 |
| **US detergent 2 + 0.25 ppm DNase** | 0,061 | 0,032 | 0,019 | 0,013 |
| **US detergent 2 + 0.5 ppm DNase** | 0,057 | 0,020 | 0,006 | 0,009 |
| **US detergent 2 + 1 ppm DNase** | 0,056 | 0,014 | 0,010 | 0,002 |
| **US detergent 2 + 2.5 ppm DNase** | 0,034 | 0,008 | 0,009 | 0,008 |
| **US detergent 2 + 5 ppm DNase** | 0,023 | 0,009 | 0,009 | 0,017 |

## Claims

1. A method for cleaning a medical device comprising the steps of:

   a) Providing a wash liquor having a pH in the range of 6.0-10.0, comprising a polypeptide having DNase activity and optionally further cleaning components,
   b) Subjecting at least part of the medical device to the wash liquor of step a),
   c) Optionally, subjecting the medical device to rinsing,
   d) Optionally, subjecting the medical device to drying, and
   e) Optionally, subjecting the medical device to sterile packaging.

2. The method according to claim 1, wherein at least a part of the medical device comprises organic soil.

3. The method according to any of the preceding claims, wherein the wash liquor comprising a polypeptide having DNase activity releases and/or removes at least part of the organic soil present on the part of the medical device subjected to the wash liquor.

4. The method according to any of the preceding claims, wherein the medical device is disinfected before being subjected to the wash liquor.

5. The method according to any of the preceding claims, wherein at least part of the medical device is subjected to a liquid solution comprising peracetic acid having a pH of 3, whereafter no live microorganisms are comprised in the organic soil.

6. The method according to any of the preceding claims, wherein the wash liquor comprising a polypeptide having DNase activity releases and/or removes at least 50% of the organic soil present on the part of the medical device subjected to the wash liquor as measured in Example 2, preferably at least 60% of the organic soil is released and/or removed, more preferably at least 70% of the organic soil is released and/or removed, even more preferably at least 80% of the organic soil is released and/or removed, most preferably at least 90% of the organic soil is released and/or removed, such as at least 95% of the organic soil is released and/or removed.

7. The method according to any of the preceding claims, wherein the polypeptide having DNase activity is of bacterial or fungal origin.

8. The method according to any of the preceding claims, wherein the polypeptide having DNase activity is selected from the group consisting of:

   a) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 1;
   b) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 2;
   c) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 3;
   d) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 4;
   e) a polypeptide having at least 65%, at least 70%, at least 75%, at least 80%, at least 85%, at least 90%, at

least 91%, at least 92%, at least 93%, at least 94%, at least 95%, at least 96%, at least 97%, at least 98%, at least 99%, or 100% sequence identity to the polypeptide shown in SEQ ID NO: 5;
f) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 6;
g) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 7;
h) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 8;
i) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 9 and
j) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 10.

**9.** A medical cleaning composition comprising a polypeptide having DNase activity, wherein the composition has a pH in the range of 6.0-10.0.

**10.** A medical cleaning composition according to claim 9, wherein the polypeptide is selected from the group consisting of:

a) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 1;
b) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 2;
c) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 3;
d) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 4;
e) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 5;
f) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 6;
g) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 7;
h) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 8;
i) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 9 and
j) a polypeptide having at least 65%, at least 70%, at least 75%, at least 80%, at least 85%, at least 90%, at least 91%, at least 92%, at least 93%, at least 94%, at least 95%, at least 96%, at least 97%, at least 98%, at least 99%, or 100% sequence identity to the polypeptide shown in SEQ ID NO: 10.

**11.** The medical cleaning composition according to any of claims 9-10, wherein the composition further comprises one or more cleaning components, preferably selected from surfactants, builders, bleach components, polymers, dispersing agents and additional enzymes.

**12.** The medical cleaning composition according to any of claims 9-11, wherein the medical cleaning composition further comprises one or more enzymes selected from the group consisting of proteases, amylases, lipases, cutinases, cellulases, endoglucanases, xyloglucanases, pectinases, pectin lyases, xanthanases, peroxidaes, haloperoxygenases, catalases, galactanase, mannanases, or any mixture thereof.

**13.** The medical cleaning composition according to any of claims 9-12 comprising 5-25% of a non-ionic surfactant and 0-5% of an anionic surfactant.

**14.** Use of a polypeptide having DNase activity for releasing and/or removing organic soil from a medical device.

**15.** Medical device coated with a composition comprising a polypeptide having DNase activity.

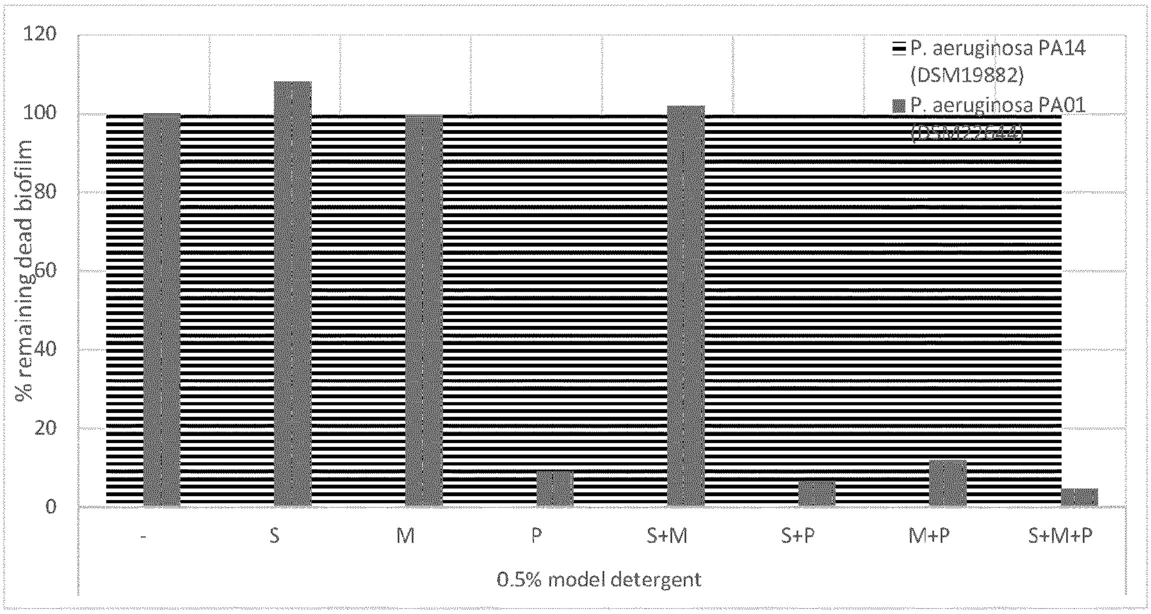


Figure 1: %remaining dead biofilm after 60 minutes treatment with 0.5% model detergent ± enzymes. -: no enzymes.

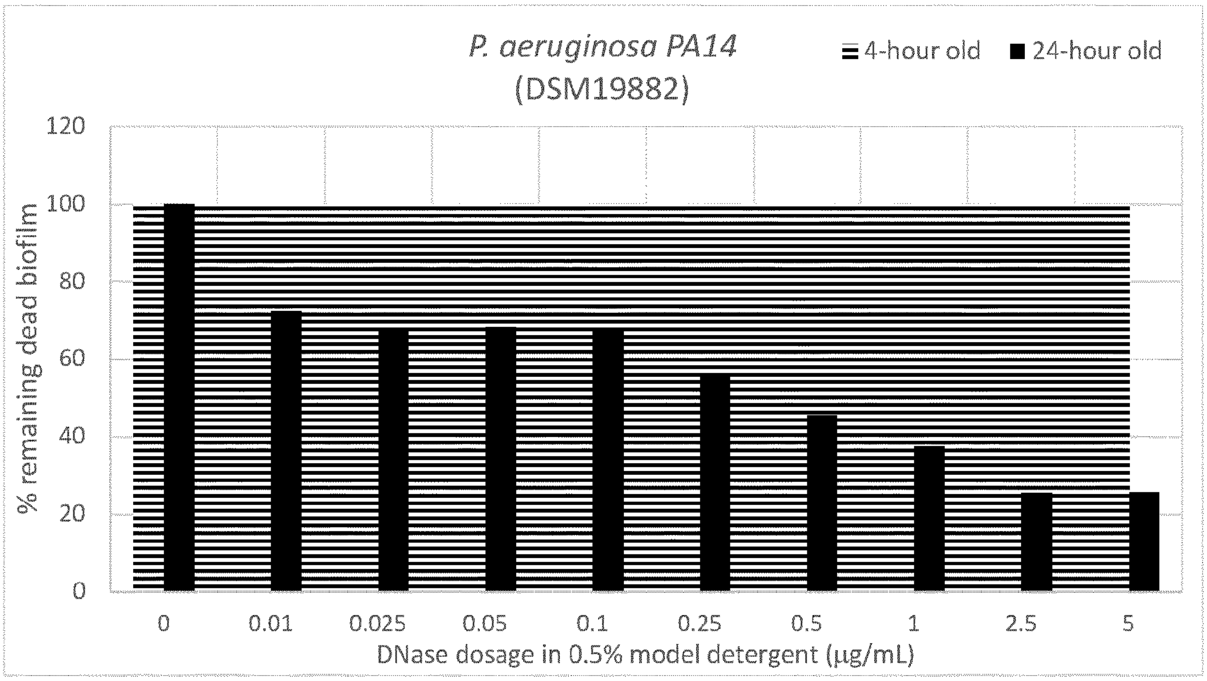


Figure 2: %remaining dead biofilm from *P. aeruginosa* PA14 (grown for 4-hours or 24-hours before kill) after 60 minutes treatment with 0.5% model detergent ± DNase in dosages from 0 microns/mL to 5 microns/mL.

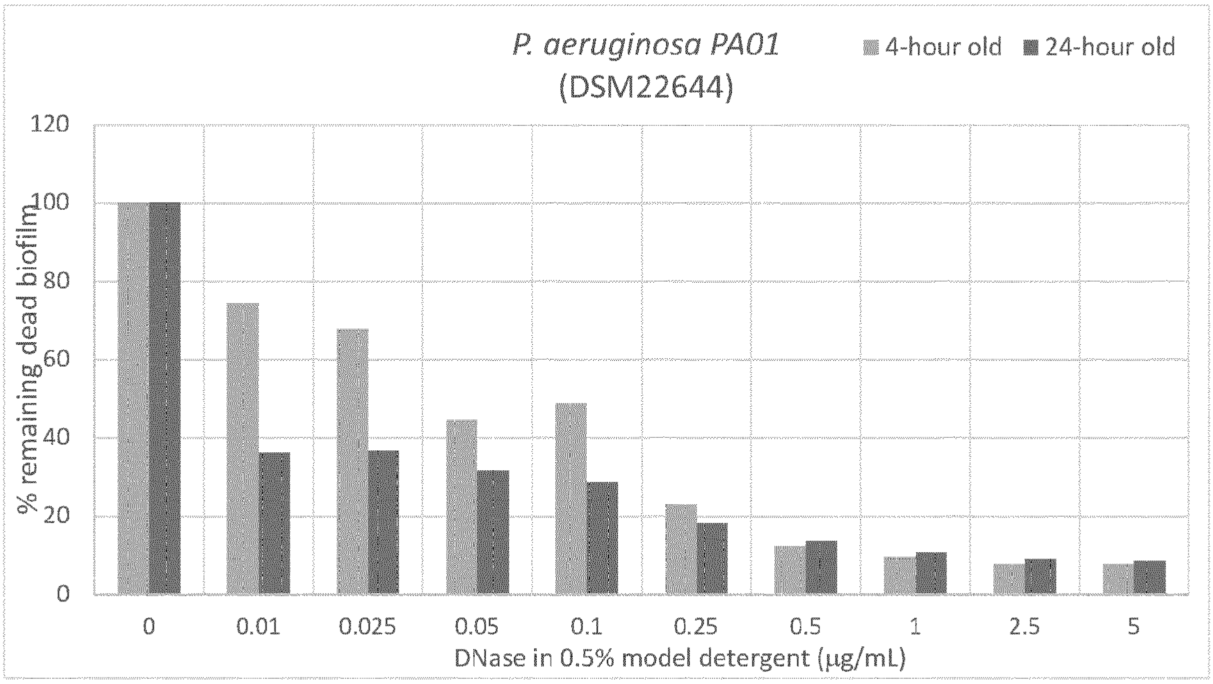


Figure 3: %remaining dead biofilm from *P. aeruginosa* PA01 (grown for 4-hours or 24-hours before kill) after 60 minutes treatment with 0.5% model detergent ± DNase in dosages from 0 microns/mL to 5 microns/mL. Left hand columns: 4 hour, Right hand columns: 24 hours.

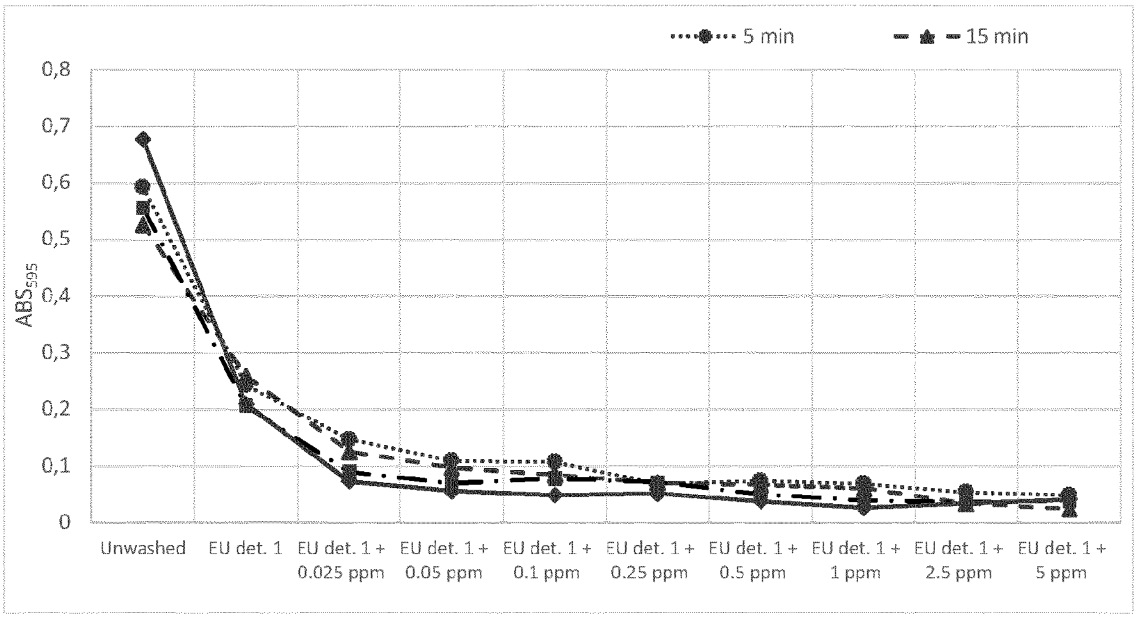


Figure 4: Absorbance of crystal violet stained dead biofilm from *P. aeruginosa* PA14 (grown for 4-hours before kill) after treatment with DNase in dosages from 0 - 5 ppm in endoscope reprocessing EU detergent 1, Dr. Weigert Neodisher Multizym.

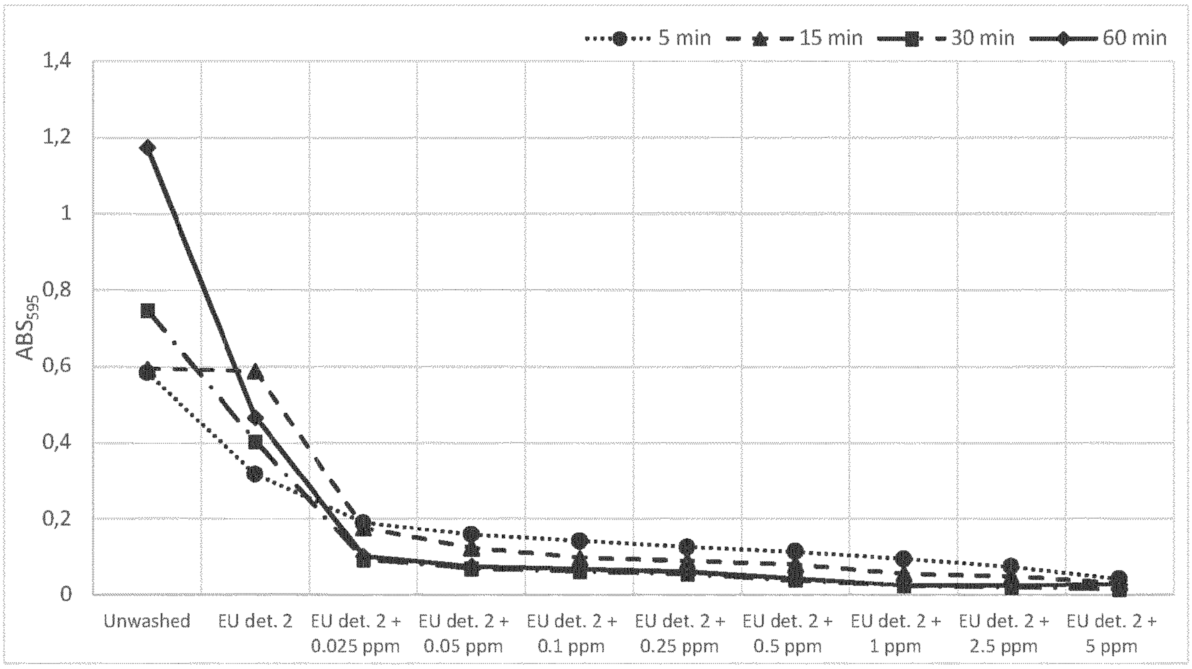


Figure 5: Absorbance of crystal violet stained dead biofilm from *P. aeruginosa* PA14 (grown for 4-hours before kill) after treatment with DNase in dosages from 0 - 5 ppm in endoscope reprocessing EU detergent 2, Aniosyme Synergy 5.

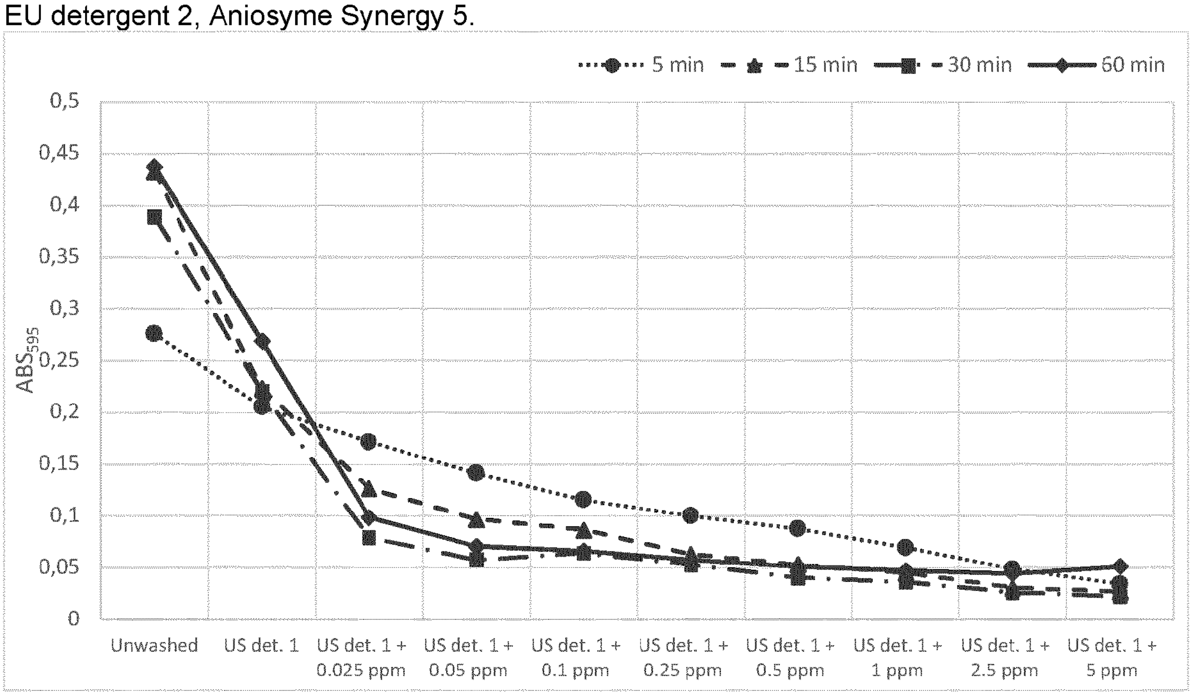


Figure 6: Absorbance of crystal violet stained dead biofilm from *P. aeruginosa* PA14 (grown for 4-hours before kill) after treatment with DNase in dosages from 0 - 5 ppm in endoscope reprocessing US detergent 1, Steris Prolystica 2X Enzymatic Presoak and Cleaner.

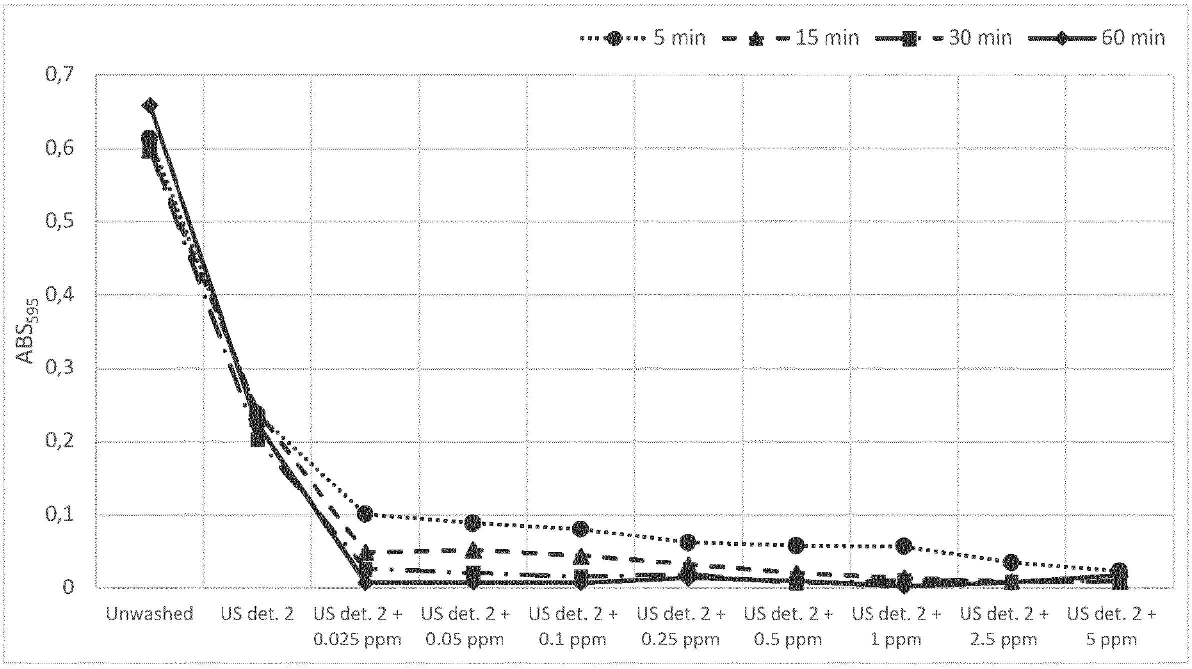


Figure 7: Absorbance of crystal violet stained dead biofilm from *P. aeruginosa* PA14 (grown for 4-hours before kill) after treatment with DNase in dosages from 0 - 5 ppm in endoscope reprocessing US detergent 2, Metrex Metrizyme.

Europäisches Patentamt
European Patent Office
Office européen des brevets

# EUROPEAN SEARCH REPORT

Application Number
EP 20 17 3450

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/098579 A1 (UNIV NEWCASTLE [GB]; BURGESS JAMES GRANT [GB] ET AL.) 18 August 2011 (2011-08-18) * paragraphs [0098], [0099], [0126], [0161]; claims 1-35; figures 1-13; examples 1-5 * ----- | 1-15 | INV. C11D3/386 |
| X | WO 2017/059802 A1 (NOVOZYMES AS [DK]; SUN TIANQI [CN]) 13 April 2017 (2017-04-13) * page 112, line 23 - page 113, line 9; sequence 21 * * page 119, line 26 - page 120, line 1 * ----- | 1,9,14, 15 | |
| A,D | WO 2017/129331 A1 (NOVOZYMES AS [DK]) 3 August 2017 (2017-08-03) * claims 1-15; examples 1-5 * ----- | 1-15 | |
| A | TETZ VICTOR V ET AL: "Effect of extracellular DNA destruction by DNase I on characteristics of forming biofilms", DNA AND CELL BIOLOGY, MARY ANN LIEBERT, NEW YORK, NY, US, vol. 29, no. 8, 1 August 2010 (2010-08-01), pages 399-405, XP009155541, ISSN: 1044-5498, DOI: 10.1089/DNA.2009.1011 [retrieved on 2010-05-22] * abstract * ----- | 1-15 | TECHNICAL FIELDS SEARCHED (IPC): C11D |

The present search report has been drawn up for all claims

1

| Place of search | Date of completion of the search | Examiner |
|---|---|---|
| The Hague | 24 September 2020 | van Klompenburg, Wim |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO. EP 20 17 3450

This annex lists the patent family members relating to the patent documents cited in the above-mentioned European search report. The members are as contained in the European Patent Office EDP file on
The European Patent Office is in no way liable for these particulars which are merely given for the purpose of information.

24-09-2020

| Patent document cited in search report | Publication date | Patent family member(s) | Publication date |
|---|---|---|---|
| WO 2011098579 A1 | 18-08-2011 | DK 2533801 T3 | 06-11-2017 |
| | | EP 2533801 A1 | 19-12-2012 |
| | | EP 3241559 A1 | 08-11-2017 |
| | | ES 2645376 T3 | 05-12-2017 |
| | | GB 2477914 A | 24-08-2011 |
| | | US 2013052250 A1 | 28-02-2013 |
| | | US 2017333601 A1 | 23-11-2017 |
| | | US 2017340779 A1 | 30-11-2017 |
| | | WO 2011098579 A1 | 18-08-2011 |
| WO 2017059802 A1 | 13-04-2017 | BR 112018007017 A2 | 16-10-2018 |
| | | CA 2994356 A1 | 13-04-2017 |
| | | CN 108350441 A | 31-07-2018 |
| | | CN 108368154 A | 03-08-2018 |
| | | CN 108473537 A | 31-08-2018 |
| | | EP 3359657 A1 | 15-08-2018 |
| | | EP 3359658 A2 | 15-08-2018 |
| | | EP 3359659 A1 | 15-08-2018 |
| | | EP 3708660 A2 | 16-09-2020 |
| | | JP 2018535664 A | 06-12-2018 |
| | | US 2018187175 A1 | 05-07-2018 |
| | | US 2019055528 A1 | 21-02-2019 |
| | | US 2020123476 A1 | 23-04-2020 |
| | | WO 2017059801 A1 | 13-04-2017 |
| | | WO 2017059802 A1 | 13-04-2017 |
| | | WO 2017060475 A2 | 13-04-2017 |
| | | WO 2017060493 A1 | 13-04-2017 |
| | | WO 2017060505 A1 | 13-04-2017 |
| WO 2017129331 A1 | 03-08-2017 | CN 108603146 A | 28-09-2018 |
| | | EP 3408366 A1 | 05-12-2018 |
| | | US 2019024022 A1 | 24-01-2019 |
| | | WO 2017129331 A1 | 03-08-2017 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017129331 A
- WO 2014110015 A
- WO 8906279 A
- WO 9318140 A
- WO 98020115 A
- WO 0144452 A
- WO 0158275 A
- WO 0158276 A
- WO 03006602 A
- WO 04099401 A
- WO 9219729 A
- WO 9820115 A
- WO 9820116 A
- WO 9834946 A
- WO 99064619 A
- EP 258068 A
- EP 305216 A
- WO 9613580 A
- EP 218272 A
- EP 331376 A
- GB 1372034 A
- WO 9506720 A
- WO 9627002 A
- WO 9612012 A
- JP 64744992 B
- WO 9116422 A
- WO 10065455 A
- WO 10107560 A
- US 5389536 A
- WO 11084412 A
- WO 11084417 A
- WO 11084599 A
- WO 11150157 A
- WO 12137147 A
- WO 9205249 A
- WO 9401541 A
- EP 407225 A
- EP 260105 A
- WO 9535381 A
- WO 9600292 A
- WO 9530744 A
- WO 9425578 A
- WO 9514783 A
- WO 9522615 A
- WO 9704079 A
- WO 9707202 A
- WO 00060063 A
- WO 07087508 A
- WO 09109500 A
- GB 1296839 A
- WO 9510603 A
- WO 9402597 A
- WO 9418314 A
- WO 9743424 A
- WO 99019467 A
- WO 02010355 A
- WO 2006066594 A
- WO 96023873 A
- WO 08153815 A
- WO 0166712 A
- WO 09061380 A
- WO 2011098531 A
- WO 2013001078 A
- WO 2013001087 A
- US 6124127 A
- WO 99027083 A
- WO 99027084 A
- WO 02006442 A
- WO 02092741 A
- WO 03095638 A
- US 4435307 A
- US 5648263 A
- US 5691178 A
- US 5776757 A
- WO 8909259 A
- EP 0495257 A
- EP 0531372 A
- WO 9611262 A
- WO 9629397 A
- WO 9808940 A
- WO 9407998 A
- EP 0531315 A
- US 5457046 A
- US 5686593 A
- EP 179486 A
- WO 9324618 A
- WO 9510602 A
- WO 9815257 A
- WO 9527046 A
- WO 9704102 A
- WO 0179459 A
- WO 0179458 A
- WO 0179461 A
- WO 0179460 A
- WO 9201046 A
- JP 2238885 A
- WO 9708325 A
- WO 9533836 A

- WO 2005056782 A
- WO 2008063400 A
- US 2008145353 A
- US 2007167344 A
- WO 09102854 A
- US 5977053 A
- WO 2006130575 A
- US 5955415 A
- US 5275753 A
- WO 9900478 A
- WO 9817767 A
- EP 624154 A
- WO 2007087258 A
- WO 2007087244 A
- WO 2007087259 A
- WO 2007087242 A


**Non-patent literature cited in the description**


- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- Class II Special Controls Guidance Document: Medical Washers and Medical Washer-Disinfectors; Guidance for the Medical Device Industry and FDA Review Staff. *U.S. Food and Drug Administration,* February 2002
- **SIEZEN et al.** *Protein Engng.,* 1991, vol. 4, 719-737
- **SIEZEN et al.** *Protein Science,* 1997, vol. 6, 501-523
- **DARTOIS et al.** *Biochemica et Biophysica Acta,* 1993, vol. 1131, 253-360
- **HODGDON ; KALER.** *Current Opinion in Colloid & Interface Science,* 2007, vol. 12, 121-128

SEQUENCE LISTING

<110> Novozymes A/S

<120> MEDICAL CLEANING COMPOSITION, USE AND METHOD OF CLEANING

<130> 15186-EP-EPA

<160> 13

<170> PatentIn version 3.5

<210> 1
<211> 182
<212> PRT
<213> Bacillus cibi

<400> 1

Thr Pro Pro Gly Thr Pro Ser Lys Ser Ala Ala Gln Ser Gln Leu Asn
1 5 10 15

Ala Leu Thr Val Lys Thr Glu Gly Ser Met Ser Gly Tyr Ser Arg Asp
20 25 30

Leu Phe Pro His Trp Ile Ser Gln Gly Ser Gly Cys Asp Thr Arg Gln
35 40 45

Val Val Leu Lys Arg Asp Ala Asp Ser Tyr Ser Gly Asn Cys Pro Val
50 55 60

Thr Ser Gly Ser Trp Tyr Ser Tyr Tyr Asp Gly Val Thr Phe Thr Asn
65 70 75 80

Pro Ser Asp Leu Asp Ile Asp His Ile Val Pro Leu Ala Glu Ala Trp
85 90 95

Arg Ser Gly Ala Ser Ser Trp Thr Thr Ser Lys Arg Gln Asp Phe Ala
100 105 110

Asn Asp Leu Ser Gly Pro Gln Leu Ile Ala Val Ser Ala Ser Thr Asn
115 120 125

Arg Ser Lys Gly Asp Gln Asp Pro Ser Thr Trp Gln Pro Pro Arg Ser
130 135 140

Gly Ala Ala Cys Gly Tyr Ser Lys Trp Trp Ile Ser Thr Lys Tyr Lys
145 150 155 160

Trp Gly Leu Ser Leu Gln Ser Ser Glu Lys Thr Ala Leu Gln Gly Met
165 170 175

Leu Asn Ser Cys Ser Tyr
180

<210> 2
<211> 182
<212> PRT
<213> Artificial

<220>
<223> Artificial

<400> 2

Ile Pro Pro Gly Thr Pro Ser Lys Ser Ala Ala Gln Tyr Gln Leu Asn
1 5 10 15

Ala Leu Thr Val Lys Pro Glu Gly Ser Met Leu Gly Tyr Ser Arg Asp
20 25 30

Lys Phe Pro His Trp Ile Pro Gln Gly Gly Gly Cys Asp Thr Arg Gln
35 40 45

Val Val Leu Lys Arg Asp Ala Ile Trp Tyr Val Gly Asn Cys Pro Val
50 55 60

Val Ser Gly Ser Trp Tyr Ser Tyr Tyr Asp Gly Leu Thr Phe Thr Asn
65 70 75 80

Pro Ser Asp Leu Asp Ile Asp His Ile Val Pro Leu Ala Glu Ala Trp
85 90 95

Arg Ser Gly Ala Ser Ser Trp Thr Thr Ser Lys Arg Arg Asp Phe Ala
100 105 110

Asn Asp Leu Asp Gly Pro Gln Leu Ile Ala Val Ser Ala Ser Val Asn
115 120 125

Arg Ser Lys Gly Asp Gln Asp Pro Ser Thr Trp Gln Pro Pro Arg Pro
130 135 140

Gly Ala His Cys Gly Tyr Ser Lys Trp Trp Ile Ser Thr Lys Tyr Lys
145 150 155 160

Trp Gly Leu Ser Leu Gln Leu Ser Glu Lys Thr Ala Leu Gln Asp Met
165 170 175

Leu Asn Ser Cys Ser Tyr
180

<210> 3

<211> 182
<212> PRT
<213> Artificial

<220>
<223> Artificial

<400> 3

```
Ile Pro Pro Gly Thr Pro Ser Lys Ser Ala Ala Gln Ser Gln Leu Asn
1               5                   10                  15

Ala Leu Thr Val Lys Pro Glu Gly Ser Met Ser Gly Tyr Ser Arg Asp
            20                  25                  30

Leu Phe Pro His Trp Ile Ser Gln Gly Ser Gly Cys Asp Thr Arg Gln
        35                  40                  45

Val Val Leu Lys Arg Asp Ala Asp Trp Tyr Ser Gly Asn Cys Pro Val
    50                  55                  60

Thr Ser Gly Ser Trp Tyr Ser Tyr Tyr Asp Gly Leu Thr Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Gln Asp Phe Ala
            100                 105                 110

Asn Asp Leu Ser Gly Pro Gln Leu Ile Ala Val Ser Ala Ser Thr Asn
        115                 120                 125

Arg Ser Lys Gly Asp Gln Asp Pro Ser Thr Trp Gln Pro Pro Arg Ser
    130                 135                 140

Gly Ala His Cys Gly Tyr Ser Lys Trp Trp Ile Ser Thr Lys Tyr Lys
145                 150                 155                 160

Trp Gly Leu Ser Leu Gln Leu Ser Glu Lys Thr Ala Leu Gln Asp Met
                165                 170                 175

Leu Asn Ser Cys Ser Tyr
            180
```

<210> 4
<211> 182
<212> PRT
<213> Artificial

<220>

<223> Artificial

<400> 4

Ile Pro Pro Gly Thr Pro Ser Lys Ser Ala Ala Gln Tyr Gln Leu Asn
1               5                   10                  15

Ala Leu Thr Val Lys Pro Glu Gly Ser Met Leu Gly Tyr Ser Arg Asp
            20                  25                  30

Leu Phe Pro His Trp Ile Ser Gln Gly Gly Gly Cys Asp Thr Arg Gln
        35                  40                  45

Val Val Leu Lys Arg Asp Ala Asp Trp Tyr Val Gly Asn Cys Pro Val
    50                  55                  60

Thr Ser Gly Ser Trp Tyr Ser Tyr Tyr Asp Gly Leu Thr Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Arg Asp Phe Ala
            100                 105                 110

Asn Asp Leu Asp Gly Pro Gln Leu Ile Ala Val Ser Ala Ser Val Asn
        115                 120                 125

Arg Ser Lys Gly Asp Gln Asp Pro Ser Thr Trp Gln Pro Pro Arg Pro
    130                 135                 140

Gly Ala His Cys Gly Tyr Ser Lys Trp Trp Ile Ser Thr Lys Tyr Lys
145                 150                 155                 160

Trp Gly Leu Ser Leu Gln Leu Ser Glu Lys Thr Ala Leu Gln Asp Met
                165                 170                 175

Leu Asn Ser Cys Ser Tyr
            180

<210> 5
<211> 182
<212> PRT
<213> Artificial

<220>
<223> Artificial

<400> 5

Ile Pro Pro Gly Thr Pro Ser Lys Ser Ala Ala Gln Tyr Gln Leu Asn

1 5 10 15

Ala Leu Thr Val Lys Pro Glu Gly Pro Met Leu Gly Tyr Ser Arg Asp
20 25 30

Leu Phe Pro His Trp Ile Pro Gln Gly Gly Gly Cys Asp Thr Arg Gln
35 40 45

Val Val Leu Lys Arg Asp Ala Asp Trp Tyr Val Gly Asn Cys Pro Val
50 55 60

Val Tyr Gly Ser Trp Tyr Ser Tyr Tyr Asp Gly Leu Tyr Phe Thr Asn
65 70 75 80

Pro Ser Asp Leu Asp Ile Asp His Ile Val Pro Leu Ala Glu Ala Trp
85 90 95

Arg Ser Gly Ala Ser Ser Trp Thr Thr Ser Lys Arg Arg Asp Phe Ala
100 105 110

Asn Asp Leu Asp Gly Pro Gln Leu Ile Ala Val Ser Ala Ser Val Asn
115 120 125

Arg Ser Lys Gly Asp Gln Asp Pro Ser Thr Trp Gln Pro Pro Arg Pro
130 135 140

Gly Ala His Cys Gly Tyr Ser Lys Trp Trp Ile Ser Thr Lys Tyr Lys
145 150 155 160

Trp Gly Leu Ser Leu Gln Leu Ser Glu Lys Thr Ala Leu Gln Asp Met
165 170 175

Leu Asn Ser Cys Ser Tyr
180

<210> 6
<211> 182
<212> PRT
<213> Artificial

<220>
<223> Artificial

<400> 6

Ile Pro Pro Gly Thr Pro Ser Lys Ser Ala Ala Gln Tyr Gln Leu Asn
1 5 10 15

Ala Leu Thr Val Lys Pro Glu Gly Ser Met Leu Gly Tyr Ser Arg Asp
20 25 30

Leu Phe Pro His Trp Ile Pro Gln Gly Gly Gly Cys Asp Thr Arg Gln
35 40 45

Val Val Leu Lys Arg Asp Ala Leu Trp Tyr Val Gly Asn Cys Pro Val
50 55 60

Val Ser Gly Ser Trp Tyr Ser Tyr Tyr Asp Gly Leu Thr Phe Thr Asn
65 70 75 80

Pro Ser Asp Leu Asp Ile Asp His Ile Val Pro Leu Ala Glu Ala Trp
85 90 95

Arg Ser Gly Ala Ser Ser Trp Thr Thr Ser Lys Arg Arg Asp Phe Ala
100 105 110

Asn Asp Leu Asp Gly Pro Gln Leu Ile Ala Val Ser Ala Ser Val Asn
115 120 125

Arg Ser Lys Gly Asp Gln Asp Pro Ser Thr Trp Gln Pro Pro Arg Pro
130 135 140

Gly Ala His Cys Gly Tyr Ser Lys Trp Trp Ile Ser Thr Lys Tyr Lys
145 150 155 160

Trp Asp Leu Ser Leu Gln Leu Ser Glu Lys Thr Ala Leu Gln Asp Met
165 170 175

Leu Asn Ser Cys Ser Tyr
180

<210> 7
<211> 182
<212> PRT
<213> Artificial

<220>
<223> Artificial

<400> 7

Ile Pro Pro Gly Thr Pro Ser Lys Ser Ala Ala Gln Tyr Gln Leu Asn
1 5 10 15

Ala Leu Thr Val Lys Pro Glu Gly Ser Met Arg Gly Tyr Ser Arg Asp
20 25 30

Leu Phe Pro His Trp Ile Pro Gln Gly Gly Gly Cys Asp Thr Arg Gln
35 40 45

```
Val Val Leu Lys Arg Asp Ala Leu Trp Tyr Val Gly Asn Cys Pro Val
    50                  55                  60

Val Ser Gly Ser Trp Tyr Ser Tyr Tyr Asp Gly Leu Thr Phe Thr Asn
65                  70                  75                  80

Pro Ser Asp Leu Asp Ile Asp His Ile Val Pro Leu Ala Glu Ala Trp
                85                  90                  95

Arg Ser Gly Ala Ser Ser Trp Thr Thr Ser Lys Arg Arg Asp Phe Ala
            100                 105                 110

Asn Asp Leu Asp Gly Pro Gln Leu Ile Ala Val Ser Ala Ser Val Asn
        115                 120                 125

Arg Ser Lys Gly Asp Gln Asp Pro Ser Thr Trp Gln Pro Pro Arg Pro
    130                 135                 140

Gly Ala His Cys Gly Tyr Ser Lys Trp Trp Ile Ser Thr Lys Tyr Lys
145                 150                 155                 160

Trp Asp Leu Ser Leu Gln Leu Ser Glu Lys Thr Ala Leu Gln Asp Met
                165                 170                 175

Leu Asn Ser Cys Ser Tyr
            180


<210>  8
<211>  204
<212>  PRT
<213>  Aspergillus oryzae

<400>  8

Lys Thr Gly Ser Gly Asp Ser Gln Ser Asp Pro Ile Lys Ala Asp Leu
1               5                   10                  15

Glu Val Lys Gly Gln Ser Ala Leu Pro Phe Asp Val Asp Cys Trp Ala
            20                  25                  30

Ile Leu Cys Lys Gly Ala Pro Asn Val Leu Gln Arg Val Asn Glu Lys
        35                  40                  45

Thr Lys Asn Ser Asn Arg Asp Arg Ser Gly Ala Asn Lys Gly Pro Phe
    50                  55                  60

Lys Asp Pro Gln Lys Trp Gly Ile Lys Ala Leu Pro Pro Lys Asn Pro
65                  70                  75                  80
```

```
Ser Trp Ser Ala Gln Asp Phe Lys Ser Pro Glu Glu Tyr Ala Phe Ala
                85                  90                  95

Ser Ser Leu Gln Gly Gly Thr Asn Ala Ile Leu Ala Pro Val Asn Leu
            100                 105                 110

Ala Ser Gln Asn Ser Gln Gly Gly Val Leu Asn Gly Phe Tyr Ser Ala
        115                 120                 125

Asn Lys Val Ala Gln Phe Asp Pro Ser Lys Pro Gln Gln Thr Lys Gly
    130                 135                 140

Thr Trp Phe Gln Ile Thr Lys Phe Thr Gly Ala Ala Gly Pro Tyr Cys
145                 150                 155                 160

Lys Ala Leu Gly Ser Asn Asp Lys Ser Val Cys Asp Lys Asn Lys Asn
                165                 170                 175

Ile Ala Gly Asp Trp Gly Phe Asp Pro Ala Lys Trp Ala Tyr Gln Tyr
            180                 185                 190

Asp Glu Lys Asn Asn Lys Phe Asn Tyr Val Gly Lys
        195                 200


<210>  9
<211>  221
<212>  PRT
<213>  Aspergillus oryzae

<400>  9

Val Pro Val Asn Pro Glu Pro Asp Ala Thr Ser Val Glu Asn Val Ala
1               5                   10                  15

Leu Lys Thr Gly Ser Gly Asp Ser Gln Ser Asp Pro Ile Lys Ala Asp
            20                  25                  30

Leu Glu Val Lys Gly Gln Ser Ala Leu Pro Phe Asp Val Asp Cys Trp
        35                  40                  45

Ala Ile Leu Cys Lys Gly Ala Pro Asn Val Leu Gln Arg Val Asn Glu
    50                  55                  60

Lys Thr Lys Asn Ser Asn Arg Asp Arg Ser Gly Ala Asn Lys Gly Pro
65                  70                  75                  80

Phe Lys Asp Pro Gln Lys Trp Gly Ile Lys Ala Leu Pro Pro Lys Asn
                85                  90                  95
```

Pro Ser Trp Ser Ala Gln Asp Phe Lys Ser Pro Glu Glu Tyr Ala Phe
            100                 105                 110

Ala Ser Ser Leu Gln Gly Gly Thr Asn Ala Ile Leu Ala Pro Val Asn
        115                 120                 125

Leu Ala Ser Gln Asn Ser Gln Gly Gly Val Leu Asn Gly Phe Tyr Ser
    130                 135                 140

Ala Asn Lys Val Ala Gln Phe Asp Pro Ser Lys Pro Gln Gln Thr Lys
145                 150                 155                 160

Gly Thr Trp Phe Gln Ile Thr Lys Phe Thr Gly Ala Ala Gly Pro Tyr
                165                 170                 175

Cys Lys Ala Leu Gly Ser Asn Asp Lys Ser Val Cys Asp Lys Asn Lys
            180                 185                 190

Asn Ile Ala Gly Asp Trp Gly Phe Asp Pro Ala Lys Trp Ala Tyr Gln
        195                 200                 205

Tyr Asp Glu Lys Asn Asn Lys Phe Asn Tyr Val Gly Lys
    210                 215                 220

<210> 10
<211> 188
<212> PRT
<213> Trichoderma harzianum

<400> 10

Ala Pro Ala Pro Met Pro Thr Pro Pro Gly Ile Pro Thr Glu Ser Ser
1               5                   10                  15

Ala Arg Thr Gln Leu Ala Gly Leu Thr Val Ala Val Ala Gly Ser Gly
            20                  25                  30

Thr Gly Tyr Ser Arg Asp Leu Phe Pro Thr Trp Asp Ala Ile Ser Gly
        35                  40                  45

Asn Cys Asn Ala Arg Glu Tyr Val Leu Lys Arg Asp Gly Glu Gly Val
    50                  55                  60

Gln Val Asn Asn Ala Cys Glu Ser Gln Ser Gly Thr Trp Ile Ser Pro
65                  70                  75                  80

Tyr Asp Asn Ala Ser Phe Thr Asn Ala Ser Ser Leu Asp Ile Asp His
                85                  90                  95

Met Val Pro Leu Lys Asn Ala Trp Ile Ser Gly Ala Ser Ser Trp Thr
100 105 110

Thr Ala Gln Arg Glu Ala Leu Ala Asn Asp Val Ser Arg Pro Gln Leu
115 120 125

Trp Ala Val Ser Ala Ser Ala Asn Arg Ser Lys Gly Asp Arg Ser Pro
130 135 140

Asp Gln Trp Lys Pro Pro Leu Thr Ser Phe Tyr Cys Thr Tyr Ala Lys
145 150 155 160

Ser Trp Ile Asp Val Lys Ser Phe Tyr Lys Leu Thr Ile Thr Ser Ala
165 170 175

Glu Lys Thr Ala Leu Ser Ser Met Leu Asp Thr Cys
180 185

<210> 11
<211> 269
<212> PRT
<213> Bacillus lentus

<400> 11

Ala Gln Ser Val Pro Trp Gly Ile Ser Arg Val Gln Ala Pro Ala Ala
1 5 10 15

His Asn Arg Gly Leu Thr Gly Ser Gly Val Lys Val Ala Val Leu Asp
20 25 30

Thr Gly Ile Ser Thr His Pro Asp Leu Asn Ile Arg Gly Gly Ala Ser
35 40 45

Phe Val Pro Gly Glu Pro Ser Thr Gln Asp Gly Asn Gly His Gly Thr
50 55 60

His Val Ala Gly Thr Ile Ala Ala Leu Asn Asn Ser Ile Gly Val Leu
65 70 75 80

Gly Val Ala Pro Ser Ala Glu Leu Tyr Ala Val Lys Val Leu Gly Ala
85 90 95

Ser Gly Ser Gly Ser Val Ser Ser Ile Ala Gln Gly Leu Glu Trp Ala
100 105 110

Gly Asn Asn Gly Met His Val Ala Asn Leu Ser Leu Gly Ser Pro Ser
115 120 125

```
Pro Ser Ala Thr Leu Glu Gln Ala Val Asn Ser Ala Thr Ser Arg Gly
    130                 135                 140

Val Leu Val Val Ala Ala Ser Gly Asn Ser Gly Ala Gly Ser Ile Ser
145                 150                 155                 160

Tyr Pro Ala Arg Tyr Ala Asn Ala Met Ala Val Gly Ala Thr Asp Gln
                165                 170                 175

Asn Asn Asn Arg Ala Ser Phe Ser Gln Tyr Gly Ala Gly Leu Asp Ile
            180                 185                 190

Val Ala Pro Gly Val Asn Val Gln Ser Thr Tyr Pro Gly Ser Thr Tyr
        195                 200                 205

Ala Ser Leu Asn Gly Thr Ser Met Ala Thr Pro His Val Ala Gly Ala
    210                 215                 220

Ala Ala Leu Val Lys Gln Lys Asn Pro Ser Trp Ser Asn Val Gln Ile
225                 230                 235                 240

Arg Asn His Leu Lys Asn Thr Ala Thr Ser Leu Gly Ser Thr Asn Leu
                245                 250                 255

Tyr Gly Ser Gly Leu Val Asn Ala Glu Ala Ala Thr Arg
            260                 265


<210>  12
<211>  275
<212>  PRT
<213>  Bacillus amyloliquefaciens

<400>  12

Ala Gln Ser Val Pro Tyr Gly Val Ser Gln Ile Lys Ala Pro Ala Leu
1               5                   10                  15

His Ser Gln Gly Tyr Thr Gly Ser Asn Val Lys Val Ala Val Ile Asp
            20                  25                  30

Ser Gly Ile Asp Ser Ser His Pro Asp Leu Lys Val Ala Gly Gly Ala
        35                  40                  45

Ser Met Val Pro Ser Glu Thr Asn Pro Phe Gln Asp Asn Asn Ser His
    50                  55                  60

Gly Thr His Val Ala Gly Thr Val Ala Ala Leu Asn Asn Ser Ile Gly
65                  70                  75                  80
```

Val Leu Gly Val Ala Pro Ser Ala Ser Leu Tyr Ala Val Lys Val Leu
85 90 95

Gly Ala Asp Gly Ser Gly Gln Tyr Ser Trp Ile Ile Asn Gly Ile Glu
100 105 110

Trp Ala Ile Ala Asn Asn Met Asp Val Ile Asn Met Ser Leu Gly Gly
115 120 125

Pro Ser Gly Ser Ala Ala Leu Lys Ala Ala Val Asp Lys Ala Val Ala
130 135 140

Ser Gly Val Val Val Val Ala Ala Ala Gly Asn Glu Gly Thr Ser Gly
145 150 155 160

Ser Ser Ser Thr Val Gly Tyr Pro Gly Lys Tyr Pro Ser Val Ile Ala
165 170 175

Val Gly Ala Val Asp Ser Ser Asn Gln Arg Ala Ser Phe Ser Ser Val
180 185 190

Gly Pro Glu Leu Asp Val Met Ala Pro Gly Val Ser Ile Gln Ser Thr
195 200 205

Leu Pro Gly Asn Lys Tyr Gly Ala Tyr Asn Gly Thr Ser Met Ala Ser
210 215 220

Pro His Val Ala Gly Ala Ala Ala Leu Ile Leu Ser Lys His Pro Asn
225 230 235 240

Trp Thr Asn Thr Gln Val Arg Ser Ser Leu Glu Asn Thr Thr Thr Lys
245 250 255

Leu Gly Asp Ser Phe Tyr Tyr Gly Lys Gly Leu Ile Asn Val Gln Ala
260 265 270

Ala Ala Gln
275

<210> 13
<211> 298
<212> PRT
<213> Bacillus bogoriensis

<400> 13

Ala Asn Ser Gly Phe Tyr Val Ser Gly Thr Thr Leu Tyr Asp Ala Asn
1 5 10 15

```
Gly Asn Pro Phe Val Met Arg Gly Ile Asn His Gly His Ala Trp Tyr
            20                  25                  30

Lys Asp Gln Ala Thr Thr Ala Ile Glu Gly Ile Ala Asn Thr Gly Ala
        35                  40                  45

Asn Thr Val Arg Ile Val Leu Ser Asp Gly Gly Gln Trp Thr Lys Asp
    50                  55                  60

Asp Ile His Thr Val Arg Asn Leu Ile Ser Leu Ala Glu Asp Asn His
65                  70                  75                  80

Leu Val Ala Val Leu Glu Val His Asp Ala Thr Gly Tyr Asp Ser Ile
                85                  90                  95

Ala Ser Leu Asn Arg Ala Val Asp Tyr Trp Ile Glu Met Arg Ser Ala
            100                 105                 110

Leu Ile Gly Lys Glu Asp Thr Val Ile Ile Asn Ile Ala Asn Glu Trp
        115                 120                 125

Phe Gly Ser Trp Glu Gly Asp Ala Trp Ala Asp Gly Tyr Lys Gln Ala
    130                 135                 140

Ile Pro Arg Leu Arg Asn Ala Gly Leu Asn His Thr Leu Met Val Asp
145                 150                 155                 160

Ala Ala Gly Trp Gly Gln Phe Pro Gln Ser Ile His Asp Tyr Gly Arg
                165                 170                 175

Glu Val Phe Asn Ala Asp Pro Gln Arg Asn Thr Met Phe Ser Ile His
            180                 185                 190

Met Tyr Glu Tyr Ala Gly Gly Asn Ala Ser Gln Val Arg Thr Asn Ile
        195                 200                 205

Asp Arg Val Leu Asn Gln Asp Leu Ala Leu Val Ile Gly Glu Phe Gly
    210                 215                 220

His Arg His Thr Asn Gly Asp Val Asp Glu Ala Thr Ile Met Ser Tyr
225                 230                 235                 240

Ser Glu Gln Arg Gly Val Gly Trp Leu Ala Trp Ser Trp Lys Gly Asn
                245                 250                 255

Gly Pro Glu Trp Glu Tyr Leu Asp Leu Ser Asn Asp Trp Ala Gly Asn
            260                 265                 270
```

```
Asn Leu Thr Ala Trp Gly Asn Thr Ile Val Asn Gly Pro Tyr Gly Leu
        275                 280                 285

Arg Glu Thr Ser Arg Leu Ser Thr Val Phe
    290                 295
```